# EUROPEAN PATENT APPLICATION

(11) **EP 3 560 945 A1**
(43) Date of publication of application: **30.10.2019**
(21) Application number: 18169762.4
(22) Date of filing: 27.04.2018
(51) Int. Cl.: C07K 1/16, C07K 1/18, C07K 1/20, C07K 1/22, C07K 16/28

(54) **METHODS FOR PURIFICATION OF POLYPEPTIDES USING POLYSORBATES**

(71) Applicant: F. Hoffmann-La Roche AG, 4070 Basel (CH)
(72) Inventor: The designation of the inventor has not yet been filed
(74) Representative: Burger, Alexander

(57) **Abstract**

Herein is reported a method for purifying a protein from a sample comprising the protein and at least one impurity with hydrolytic activity, comprising the following steps:
i)
a) applying the sample comprising the protein and at least one impurity with hydrolytic activity to a chromatography material,
b) applying a solution which comprises a polysorbate to the chromatography material, and
c) recovering the protein from the chromatography material, or

ii)
a) adding a polysorbate to the sample comprising the protein and at least one impurity with hydrolytic activity,
b) applying the mixture of step a) to a chromatography material, and
c) recovering the protein from the chromatography material, or

iii)
a) adding a polysorbate to the sample comprising the protein and at least one impurity with hydrolytic activity,
b) applying the mixture of step a) to a chromatography material,
c) applying a solution which comprises a polysorbate to the chromatography material, and
d) recovering the protein from the chromatography material.

## Description

The present invention generally relates to the field of purification of polypeptides or proteins. The present invention in particular relates to the reduction of hydrolytic activity in a sample. Herein is reported a method for purifying a polypeptide or protein by contacting a sample comprising the polypeptide with a polysorbate (e.g. Polysorbate 20 or Polysorbate 80) or a polysorbate-like surfactant before and/or during a purification step e.g. a chromatographic step.

### Background of the Invention

Surface-active agents, or surfactants, have become an indispensable component of robust and stable biopharmaceutical formulations to prevent destabilization of the active pharmaceutical ingredient (API). For this purpose, most of the currently marketed biotherapeutics like monoclonal antibodies (mABs) contain non-ionic detergents/surfactants, especially polysorbate 20 (PS20 or Tween® 20) or polysorbate 80 (PS80 or Tween® 80) [1]. It is known that polysorbates increase protein stability by minimizing the contact area of the API with surfaces [2-5] and air-liquid interfaces [5-7], and by interrupting protein-protein interactions [8, 9]. These advantageous attributes provide protection of the API against aggregation, denaturation and reduction of the effective protein concentration.

Polysorbates may have several impurities and degradants that occur in neat material as well as during quiescent storage in biopharmaceutical formulations, since polysorbates are prone to oxidation and hydrolysis leading to the formation of diverse polysorbate degradation products [1, 17, 18]. The oxidative pathway is primarily characterized by the presence of peroxides, aldehydes, ketones and short-chain esterified sorbitan/isosorbide-PEG species, while hydrolysis results in an elevated level of free fatty acids (FFA) and non-esterified sorbitan/isosorbide-PEG species. In addition, it has been reported that the appearance of visible and subvisible particulates in mAB formulations upon long-term storage is accredited to the accumulation of FFA due to their low solubility in aqueous solutions [18, 19]. The loss of molecular integrity has been associated with reduced stability of the APIs by lowering the functional surfactant concentration, which provides the protective effect, and by directly affecting the intact conformational structure [17, 20].

Hydrolysis of polysorbate can occur either enzymatically or by an acid-/base catalyzed mechanism [21]. However, the latter is negligible under typical formulation conditions. On the contrary, recent studies have unveiled difficult-to-remove CHO host cell protein (HCP) impurities, which are thought to be involved in the hydrolysis of polysorbate in biopharmaceutical formulations. It has been reported that the presence of putative phospholipase B-like 2 (PLBL2) and lipoprotein lipase (LPL) or other types of lipases and esterases may attribute to polysorbate degradation [22-24].

Recent findings establish that lipases associated with biologics manufacturing are expressed in upstream processes. In general, downstream purification processes (e.g., Protein A) are capable of removal of HCPs: however, it has been shown that some HCPs can be difficult to remove due to specific interactions with antibodies (Vanderlaan, M., et al., Bioproc Int. 2015, 13:18-29) and are thus retained in trace quantities in the drug substance and drug product (K. Lee, et al., A Chinese Hamster Ovary Cell Host Cell Protein That Impacts PS-80 Degradation. AccBio Conference (2015)). For storage of biopharmaceutical products it is therefore important to control and minimize the degradation of polysorbate and the formation of particles which are for example formed by accumulation of free fatty acids. In particular the enzymatic degradation of polysorbate needs to be addressed.

There are ongoing efforts for example to identify and remove lipases from protein drugs e.g. by engineering cells with reduced lipase expression (WO2015/095568) and efforts to find formulations with reduced polysorbate degradation (WO2017/117311) to address the problem. However, there is still a need for controlling/reducing the enzymatic degradation of surfactants like polysorbate and it remains a significant challenge in biopharmaceutical development.

### Summary of the Invention

Herein is reported a method for the purification of a protein or polypeptide (e.g. an antibody) using a polysorbate in a purification step, for example in a chromatographic step, to reduce the hydrolytic activity in a sample or preparation. Also reported are methods for producing proteins or polypeptides and protein or polypeptide preparations.

It has been found that by the methods and uses of the invention the hydrolytic activity in a sample can be reduced significantly. A sample comprising the protein to be purified and an impurity with hydrolytic activity is contacted or mixed with a non-ionic surfactant like a polysorbate either before the sample is loaded onto a chromatographic material, or during the sample is purified in a chromatographic step or both, before and during a chromatographic step. In all these situations the hydrolytic activity, e.g. resulting in polysorbate degradation, can be reduced.

When compared to a sample that is purified for example with a chromatographic step without the use of a polysorbate before and/or during the chromatographic step, it was found that the hydrolytic activity (especially with respect to polysorbate hydrolysis) is considerably lowered in a sample that is contacted or mixed with a polysorbate in the purification procedure. Thus, it was found that polysorbate degradation (by hydrolysis) in a purified sample (e.g. an antibody preparation/formulation) can be reduced by implementing a step of using indeed polysorbate as an additive in a preceding purification procedure. Without being bound by this theory, interestingly, the reduction of impurities, like an enzyme with hydrolytic activity, such as an esterase, is therefore performed/achieved by adding the substrate or target of the impurity (that may later, for example during storage, be subject to hydrolytic cleavage) in/during the purification procedure.

Thus, one aspect as reported herein is a method for purifying a protein (from a sample comprising the protein and at least one impurity with hydrolytic activity), comprising the following steps:
i)
   a) applying a sample comprising the protein and at least one impurity with hydrolytic activity to a chromatography material,
   b) applying a solution which comprises a polysorbate to the chromatography material, and
   c) recovering the protein from the chromatography material,
   or
ii)
   a) adding a polysorbate to a sample comprising the protein and at least one impurity with hydrolytic activity,
   b) applying the mixture of step a) to a chromatography material, and
   c) recovering the protein from the chromatography material,
   or
iii)
   a) adding a polysorbate to a sample comprising the protein and at least one impurity with hydrolytic activity,
   b) applying the mixture of step a) to a chromatography material,
   c) applying a (washing) solution which comprises a polysorbate to the chromatography material, and
   d) recovering the protein from the chromatography material.

One aspect as reported herein is a method for reducing the hydrolytic activity in a sample comprising a protein and at least one impurity with hydrolytic activity, comprising the following steps:
i)
   a) applying the sample comprising the protein and at least one impurity with hydrolytic activity to a chromatography material,
   b) applying a solution which comprises a polysorbate to the chromatography material, and
   c) recovering the protein from the chromatography material,
   or
ii)
   a) adding a polysorbate to the sample comprising the protein and at least one impurity with hydrolytic activity,
   b) applying the mixture of step a) to a chromatography material, and
   c) recovering the protein from the chromatography material,
   or
iii)
   a) adding a polysorbate to the sample comprising the protein and at least one impurity with hydrolytic activity,
   b) applying the mixture of step a) to a chromatography material,
   c) applying a solution which comprises a polysorbate to the chromatography material, and
   d) recovering the protein from the chromatography material.

In a further embodiment of all aspects the impurity is a hydrolase.

In a further embodiment of all aspects the impurity is an esterase.

In a further embodiment of all aspects the polysorbate is selected from the group comprising polysorbate 20, polysorbate 40, polysorbate 60, polysorbate 65 or polysorbate 80.

In a further embodiment of all aspects the polysorbate is polysorbate 20.

In a further embodiment of all aspects the polysorbate is added to a final concentration of at least about 0.001% (w/v).

In a further embodiment of all aspects the polysorbate is added to a final concentration of from about 0.001% (w/v) to about 10% (w/v).

In a further embodiment of all aspects the chromatography material is an affinity chromatography material, a cation exchange chromatography material, an anion exchange chromatography material, a hydrophobic interaction chromatography material or a mixed mode chromatography material.

In a further embodiment of all aspects the chromatography material is an affinity chromatography material or a cation exchange chromatography material.

In a further embodiment of all aspects the chromatography material is an affinity chromatography material selected from Protein A chromatography or a chromatography material with a camelid-derived single domain antibody fragment as ligand.

In a further embodiment of all aspects the affinity chromatography material is CaptureSelect FcXL or MabSelect SuRe.

In a further embodiment of all aspects the cation exchange chromatography material is SP Sepharose Fast Flow.

In a further embodiment of all aspects the protein is a monoclonal antibody.

In a further embodiment of all aspects the protein is a humanized Type II anti-CD20 antibody.

One aspect as reported herein is a liquid antibody preparation with reduced hydrolytic activity (increased polysorbate degradation stability/reduced polysorbate degradation) obtained by a method as reported herein.

One aspect as reported herein is a liquid antibody preparation with increased stability obtained by a method as reported herein.

One aspect as reported herein is a liquid antibody preparation with reduced particle formation (by free fatty acid assemblies) obtained by a method as reported herein.

### Detailed Description of the Invention

Herein is reported a method for purifying a protein by contacting a sample comprising the protein (and an impurity with hydrolytic activity) with a polysorbate, like Polysorbate 20 (PS20), before and/or during a chromatographic step.

It has been found that a protein can be purified from a sample by using a polysorbate as an additive in a purification step, such as e.g. a chromatography step. In particular, impurities with hydrolytic activity like hydrolyzing enzymes (e.g. lipases) can be reduced.

Consequently, one aspect as reported herein is a method for purifying a protein from a sample comprising the protein and at least one impurity with hydrolytic activity, comprising the following steps:
i)
   a) applying the sample comprising the protein and at least one impurity with hydrolytic activity to a chromatography material (under conditions suitable that the protein is bound by the chromatography material),
   b) applying a (washing) solution which comprises a polysorbate/a polysorbate-like non-ionic surfactant to the chromatography material (under conditions suitable that the protein is bound by the chromatography material), and
   c) recovering the protein from the chromatography material,
   or
ii)
   a) adding a polysorbate to the sample comprising the protein and at least one impurity with hydrolytic activity,
   b) applying the mixture of step a) (i.e. the solution comprising the sample comprising the protein and at least one impurity with hydrolytic activity and a polysorbate of step a)) to a chromatography material (under conditions suitable that the protein is bound by the chromatography material), and
   c) recovering the protein from the chromatography material,
   or
iii)
   a) adding a polysorbate to the sample comprising the protein and at least one impurity with hydrolytic activity,
   b) applying the mixture of step a) (i.e. the solution comprising the sample comprising the protein and at least one impurity with hydrolytic activity and a polysorbate of step a)) to a chromatography material (under conditions suitable that the protein is bound by the chromatography material),
   c) applying a (washing) solution which comprises a polysorbate to the chromatography material (under conditions suitable that the protein is bound by the chromatography material), and
   d) recovering the protein from the chromatography material.

One aspect as reported herein is a method for purifying a protein from a sample comprising the protein and at least one impurity with hydrolytic activity, comprising the following steps:
i)
   a) adding a polysorbate to the sample comprising the protein and at least one impurity with hydrolytic activity,
   b) applying the mixture of step a) (i.e. the solution comprising the sample comprising the protein and at least one impurity with hydrolytic activity and a polysorbate of step a)) to a chromatography material, and
   c) recovering the protein from the chromatography material,
   or
ii)
   a) adding a polysorbate to the sample comprising the protein and at least one impurity with hydrolytic activity,
   b) applying the mixture of step a) (i.e. the solution comprising the sample comprising the protein and at least one impurity with hydrolytic activity and a polysorbate of step a)) to a chromatography material,
   c) applying a (washing) solution which comprises a polysorbate to the chromatography material, and
   d) recovering the protein from the chromatography material.

One aspect as reported herein is a method for purifying a protein from a sample comprising the protein and at least one impurity with hydrolytic activity, comprising the following steps:
i)
   a) applying the sample comprising the protein and at least one impurity with hydrolytic activity to a chromatography material,
   b) applying a (washing) solution which comprises a polysorbate to the chromatography material, and
   c) recovering the protein from the chromatography material,
   or
ii)
   a) adding a polysorbate to the sample comprising the protein and at least one impurity with hydrolytic activity,
   b) applying the mixture of step a) (i.e. the solution comprising the sample comprising the protein and at least one impurity with hydrolytic activity and a polysorbate of step a)) to a chromatography material,
   c) applying a (washing) solution which comprises a polysorbate to the chromatography material, and
   d) recovering the protein from the chromatography material.

One aspect as reported herein is a method for purifying a protein from a sample comprising the protein and at least one impurity with hydrolytic activity, comprising the following steps:
a) applying the sample comprising the protein and at least one impurity with hydrolytic activity to a chromatography material,
b) applying a (washing) solution which comprises a polysorbate to the chromatography material, and
c) recovering the protein from the chromatography material.

One aspect as reported herein is a method for purifying a protein from a sample comprising the protein and at least one impurity with hydrolytic activity, comprising the following steps:
a) adding a polysorbate to the sample comprising the protein and at least one impurity with hydrolytic activity,
b) applying the mixture of step a) (i.e. the solution comprising the sample comprising the protein and at least one impurity with hydrolytic activity and a polysorbate of step a)) to a chromatography material, and
c) recovering the protein from the chromatography material.

One aspect as reported herein is a method for purifying a protein from a sample comprising the protein and at least one impurity with hydrolytic activity, comprising the following steps:
a) adding a polysorbate to the sample comprising the protein and at least one impurity with hydrolytic activity,
b) applying the mixture of step a) (i.e. the solution comprising the sample comprising the protein and at least one impurity with hydrolytic activity and a polysorbate of step a)) to a chromatography material,
c) applying a (washing) solution which comprises a polysorbate to the chromatography material, and
d) recovering the protein from the chromatography material.

It has been found that the hydrolytic activity in a sample can be reduced by adding the substrate or target of the impurity (that may later be subject to hydrolytic cleavage during storage) in/during the purification procedure. The hydrolytic activity may be active towards polysorbates. If the hydrolytic activity is reduced, the stability of a polysorbate is increased.

One aspect as reported herein is a method for reducing the (e.g. polysorbate) hydrolytic activity (increasing polysorbate degradation stability) in a sample comprising a protein and at least one impurity with hydrolytic activity, comprising the following steps:
i)
   a) applying the sample comprising the protein and at least one impurity with hydrolytic activity to a chromatography material,
   b) applying a (washing) solution which comprises a polysorbate to the chromatography material, and
   c) recovering the protein from the chromatography material,
   or
ii)
   a) adding a polysorbate to the sample comprising the protein and at least one impurity with hydrolytic activity,
   b) applying the mixture of step a) (i.e. the solution comprising the sample comprising the protein and at least one impurity with hydrolytic activity and a polysorbate of step a)) to a chromatography material, and
   c) recovering the protein from the chromatography material,
   or
iii)
   a) adding a polysorbate to the sample comprising the protein and at least one impurity with hydrolytic activity,
   b) applying the mixture of step a) (i.e. the solution comprising the sample comprising the protein and at least one impurity with hydrolytic activity and a polysorbate of step a)) to a chromatography material,
   c) applying a (washing) solution which comprises a polysorbate to the chromatography material, and
   d) recovering the protein from the chromatography material.

The hydrolytic activity may be attributable to hydrolytic enzymes. These can be reduced by the methods as reported herein.

One aspect as reported herein is a method for reducing the amount of hydrolytic enzymes in a sample comprising a protein and hydrolytic enzymes, comprising the following steps:
a) adding a polysorbate to the sample comprising a protein and hydrolytic enzymes before the application of the sample comprising the protein and hydrolytic enzymes to a chromatography material, and/or
b) washing with a solution comprising a polysorbate after the application of the sample comprising the protein and hydrolytic enzymes to a chromatography material.

It has been found that recombinant proteins like antibodies can be produced by the methods as reported herein e.g. with improved storage stability or reduced hydrolytic activity.

One aspect as reported herein is a method for producing a (recombinant) protein comprising the following steps:
a) cultivating a mammalian cell comprising a nucleic acid encoding the (recombinant) protein,
b) recovering the (recombinant) protein from the cell or the cultivation medium, and
c) purifying the (recombinant) protein with a method as reported herein,
and thereby producing the (recombinant) protein.

One aspect as reported herein is a method for producing an antibody comprising the following steps:
a) cultivating a mammalian cell comprising a nucleic acid encoding the antibody,
b) recovering the antibody from the cell or the cultivation medium, and
c) purifying the antibody with a method as reported herein,
and thereby producing the antibody.

It is understood that the samples comprising the protein may range from very small volumes to very large volumes and may expressly include preparative samples in large scale manufacturing of biotherapeutics.

Using the methods as reported herein, also protein or antibody preparations that have reduced hydrolytic activity and, therefore, increased stability can be prepared.

It was found that by the methods and uses reported herein, the practitioners are enabled to produce protein or antibody preparations with reduced hydrolytic activity and therefore with higher degradation stability. As polysorbates are themselves added to liquid antibody preparations to increase the stability of the protein in the preparation or formulation, likewise the methods and uses reported herein are suitable to increase the stability of liquid antibody preparations/formulations.

One aspect as reported herein is a liquid antibody preparation (comprising a hydrolytically cleavable surfactant/a polysorbate) with reduced hydrolytic activity (increased polysorbate degradation stability) obtained by a method as reported herein.

One aspect as reported herein is a liquid antibody preparation with increased stability obtained by a method as reported herein.

As particle formation in protein or antibody preparations can be promoted by assemblies of free fatty acids that may evolve from the degradation or cleavage of polysorbates, particle formation in these preparations can be reduced by lowering the hydrolytic activity with a method as reported herein.

Thus, one aspect as reported herein is a liquid antibody preparation with reduced particle formation (formed by free fatty acids) obtained by a method as reported herein.

It has been found that the use of a polysorbate before or in one or more purification steps can reduce hydrolytic activity in a sample.

One aspect as reported herein is the use of a polysorbate for reducing the hydrolytic activity (increasing polysorbate degradation stability) in a sample comprising a protein,
wherein the sample comprising a protein is
i) supplemented with a polysorbate before the application of the sample comprising the protein to a chromatography material, and/or
ii) washed with a solution comprising a polysorbate after the application of the sample comprising the protein to a chromatography material.

In the following embodiments of the aspects as reported herein are given. It is expressly stated that any combination of individual embodiments is also encompassed by this listing.

Impurities occur during pharmaceutical manufacturing processes inter alia due to the release of host cell proteins from the used host cells, like for example Chinese Hamster ovary (CHO) cells. These HCPs may have hydrolytic activity. Exemplary HCPs belong to the enzyme class of hydrolases including lipases like lipoprotein lipase (LPL) that hydrolyzes ester bonds within triglycerides.

An "impurity with hydrolytic activity" or "hydrolytically active impurity" (used interchangeably herein), are capable of hydrolyzing chemical bonds, i.e. cleaving chemical bonds by hydrolysis, i.e. by the addition of water. One example of acid-base hydrolysis is the hydrolysis of amides or esters. The hydrolysis occurs when the nucleophile attacks the carbon of the carbonyl group of the ester or amide. Hydrolysis may be catalyzed by enzymes like proteases or esterases.

In one embodiment the impurity (with hydrolytic activity) is an enzyme. In a further embodiment the impurity is a hydrolase. In a further embodiment the impurity is an esterase. In a further embodiment the impurity is a lipase.

A "polysorbate" is a substance derived from ethoxylated sorbitan (a derivative of sorbitol) esterified with fatty acids (i.e. a polyoxyethylene sorbitan ester). Polysorbates are a class of emulsifiers (also known as surfactants) and polysorbates are non-ionic surfactants. Polysorbates are hydrolytically cleavable surfactants. Examples of polysorbates are e.g. polysorbate 20 (polyoxyethylene (20) sorbitan monolaurate), polysorbate 40 (polyoxyethylene (20) sorbitan monopalmitate), polysorbate 60 (polyoxyethylene (20) sorbitan monostearate), polysorbate 65 (polyoxyethylene (20) sorbitan tristearate), polysorbate 80 (polyoxyethylene (20) sorbitan monooleate). The number 20 following the 'polyoxyethylene' refers to the total number of oxyethylene -(CH2CH2O)- groups found in the molecule. The number following the 'polysorbate' is related to the type of fatty acid associated with the polyoxyethylene sorbitan part of the molecule. Monolaurate is indicated by 20, monopalmitate is indicated by 40, monostearate by 60, and monooleate by 80. Common brand names for polysorbates include Scattics, Alkest, Canarcel, and Tween; e.g. Tween 20 for polysorbate 20. A "Polysorbate-like non-ionic surfactant" is a surfactant that shows similar chemical characteristics or behavior as a polysorbate.

In a further embodiment the polysorbate is selected from the group comprising polysorbate 20, polysorbate 40, polysorbate 60, polysorbate 61, polysorbate 65, polysorbate 80, polysorbate 81, polysorbate 85 or polysorbate 120, or a combination thereof. In a further embodiment the polysorbate is selected from the group comprising polysorbate 20, polysorbate 40, polysorbate 60, polysorbate 65, polysorbate 80, or a combination thereof. In a further embodiment the polysorbate is selected from the group comprising polysorbate 20 or polysorbate 80, or a combination thereof. In a further embodiment the polysorbate is polysorbate 20. In a further embodiment the polysorbate is polysorbate 80.

In a further embodiment the polysorbate is added to a final concentration of at least about 0.001% (w/v) and/or the washing solution comprising a polysorbate has a final concentration of at least about 0.001% (w/v). In a further embodiment the polysorbate is added to a final concentration of at least about 0.005% (w/v) and/or the washing solution comprising a polysorbate has a final concentration of at least about 0.005% (w/v). In a further embodiment the polysorbate is added to a final concentration of at least about 0.007% (w/v) and/or the washing solution comprising a polysorbate has a final concentration of at least about 0.007% (w/v). In a further embodiment the polysorbate is added to a final concentration of at least about 0.01% (w/v) and/or the washing solution comprising a polysorbate has a final concentration of at least about 0.01% (w/v). In a further embodiment the polysorbate is added to a final concentration of at least about 0.04% (w/v) and/or the washing solution comprising a polysorbate has a final concentration of at least about 0.04% (w/v). In a further embodiment the polysorbate is added to a final concentration of from about 0.001% (w/v) to about 10% (w/v) and/or the washing solution comprising a polysorbate has a final concentration of at least about 0.001% (w/v) to about 10% (w/v). In a further embodiment the polysorbate is added to a final concentration of from about 0.001% (w/v) to about 5% (w/v) and/or the washing solution comprising a polysorbate has a final concentration of at least about 0.001% (w/v) to about 5% (w/v). In a further embodiment the polysorbate is added to a final concentration of from about 0.001% (w/v) to about 3% (w/v) and/or the washing solution comprising a polysorbate has a final concentration of at least about 0.001% (w/v) to about 3% (w/v). In a further embodiment the polysorbate is added to a final concentration of from about 0.001 % (w/v) to about 2% (w/v) and/or the washing solution comprising a polysorbate has a final concentration of at least about 0.001% (w/v) to about 2% (w/v). In a further embodiment the polysorbate is added to a final concentration of from about 0.001 % (w/v) to about 1% (w/v) and/or the washing solution comprising a polysorbate has a final concentration of at least about 0.001% (w/v) to about 1% (w/v). It is understood that after adding a polysorbate to the sample comprising the protein/polypeptide and at least one impurity with hydrolytic activity, the mixture is incubated for a certain amount of time. The same may be done when using a polysorbate containing solution in a wash step e.g. by washing with a low flow rate or stopping the flow for a certain amount of time.

General chromatographic methods and their use are known to a person skilled in the art. See for example, Heftmann, E., (ed.), Chromatography, 5th edition, Part A: Fundamentals and Techniques, Elsevier Science Publishing Company, New York (1992); Deyl, Z., (ed.), Advanced Chromatographic and Electromigration Methods in Biosciences, Elsevier Science BV, Amsterdam, The Netherlands (1998); Poole, C.F., and Poole, S.K., Chromatography Today, Elsevier Science Publishing Company, New York (1991); Scopes, Protein Purification: Principles and Practice, Springer Verlag (1982); Sambrook, J., et al., (eds.), Molecular Cloning: A Laboratory Manual, Second Edition, Cold Spring Harbor Laboratory Press, Cold Spring Harbor, N.Y., (1989); or Ausubel, F.M., et al., (eds.), Current Protocols in Molecular Biology, John Wiley & Sons, Inc., New York (1987-1994).

Different methods are well established and widespread used for protein recovery and purification, such as affinity chromatography with microbial proteins (e.g. protein A or protein G or protein L affinity chromatography) affinity chromatography with a recombinant protein as ligand (e.g. single chain Fv as ligand, e.g. Kappa select), affinity chromatography with a camelid-derived single domain antibody fragment as ligand (e.g. Capture Select FcXL), ion exchange chromatography (e.g. cation exchange (carboxymethyl resins), anion exchange (amino ethyl resins) and mixed-mode exchange), thiophilic adsorption (e.g. with beta-mercaptoethanol and other SH ligands), hydrophobic interaction or aromatic adsorption chromatography (e.g. with phenyl-sepharose, aza-arenophilic resins, or m-aminophenylboronic acid), metal chelate affinity chromatography (e.g. with Ni(II)- and Cu(II)-affinity material), size exclusion chromatography, and electrophoretical methods (such as gel electrophoresis, capillary electrophoresis).

These methods can be combined independently in different embodiments as reported herein.

The term "applying to" denotes a partial step of a purification method in which a solution is brought in contact with a chromatography or chromatographic (terms can be used interchangeably) material. This denotes that either a) the solution is added to a chromatographic device in which the chromatography material is contained, or b) that the chromatography material is added to the solution. In case a) the solution passes through the device allowing for an interaction between the chromatography material and the substances contained in the solution. Depending on the conditions, such as e.g. pH, conductivity, salt concentration, temperature, and/or flow rate, some substances of the solution bind to the chromatography material and thus can be recovered from the chromatography material in a further step. The substances remaining in solution can be found in the flow-through. The "flow-through" denotes the solution obtained after the passage of the device, which may either be the applied solution or a buffered solution, which is used to wash the column or to cause elution of substances bound to the chromatography material.

The skilled person understands how to recover a protein from a given chromatography material. For example this may be by applying a solution with low pH value or increased salt concentration so that the protein is no longer bound by the chromatography material and is found in this solution (e.g. an elution fraction obtained by applying an elution buffer).

The device can be for example a column or a cassette. In case b) the chromatography material can be added, e.g. as a solid, to the solution, e.g. containing the substance of interest to be purified, allowing for an interaction between the chromatography material and the substances in solution. After the interaction the chromatography material is removed, e.g. by filtration, and substance bound to the chromatography material are also removed therewith from the solution, whereas substances not bound to the chromatography material remain in solution. In the cases where a (washing) solution comprising a polysorbate is implemented as one step of the procedure, it is understood that the conditions of the chromatography step are chosen that way that the protein of interest remains bound to the chromatography material.

A chromatographic "material" as used herein, such as a cation exchange material or an affinity material, refers to the stationary phase or solid phase. This may also be referred to as a resin or matrix. The "material" in this context provides a matrix to which a component of a mixture, such as protein of interest, may bind. The material may be or may comprise a column, for example an expanded bed or packed bed column. The material may be in the form of discrete particles or beads. The material may in the form of a membrane. The material may be in the form of a porous monolithic material. The material may be in the form of a functionalized fiber, fleece, or mesh. The material may be in the form of any other solid support able to carry functional groups or exhibiting chromatographic properties. A ligand capable of interacting with the protein to be purified is covalently bound to the solid phase. For example, "protein A chromatographic material" denotes an inert solid phase to which a protein A is covalently bound.

In a further embodiment the chromatography material is an affinity chromatography material, a cation exchange chromatography material, an anion exchange chromatography material, a hydrophobic interaction chromatography material or a mixed mode chromatography material. In a further embodiment the chromatography material is an affinity chromatography material, a cation exchange chromatography material or an anion exchange chromatography material. In a further embodiment the chromatography material is an affinity chromatography material or a cation exchange chromatography material.

In a further embodiment the chromatography material is an affinity chromatography material. In a further embodiment the chromatography material is an affinity chromatography material selected from the group comprising protein A or protein G or protein L affinity chromatography, single chain Fv ligand affinity chromatography, metal chelate affinity chromatography or an affinity chromatography material with a camelid-derived single domain antibody fragment as ligand (FcXL). In a further embodiment the chromatography material is an affinity chromatography material selected from Protein A chromatography or a chromatography material with a camelid-derived single domain antibody fragment as ligand. In a further embodiment the affinity chromatography material is a material with a matrix of aldehyde-activated agarose and a CaptureSelect FcXL affinity ligand [being a camelid-derived single domain antibody fragment] which specifically binds to the CH3 domain of all human IgG subclasses, wherein the ligand is coupled to the matrix by aldehyde coupling via NH2 residues of the ligand (CaptureSelect™ FcXL) or a material with a matrix of cross-linked agarose and an alkali-stabilized Protein A-derived ligand (MabSelect™ SuRe™). In a further embodiment the affinity chromatography material is a material with a matrix of aldehyde-activated agarose, an average particle size of 65 µm and a CaptureSelect™ FcXL affinity ligand [being a camelid-derived single domain antibody fragment] which specifically binds to the CH3 domain of all human IgG subclasses, wherein the ligand is coupled to the matrix by aldehyde coupling via NH2 residues of the ligand (CaptureSelect™ FcXL) or a material with a matrix of cross-linked agarose, an alkali-stabilized Protein A-derived ligand and an average particle size of 85 µm (MabSelect™ SuRe™). In a further embodiment the affinity chromatography material is CaptureSelect™ FcXL or MabSelect™ SuRe™.

In a further embodiment the chromatography material is a cation exchange chromatography material. In a further embodiment the cation exchange chromatography material is a material with a matrix of cross-linked agarose and a sulphopropyl as ligand (SP Sepharose® Fast Flow). In a further embodiment the cation exchange chromatography material is a material with a matrix of cross-linked agarose (6%), with a particle size of 45 to 165 µm (average 90 µm) and a sulphopropyl as ligand (SP Sepharose® Fast Flow). In a further embodiment the cation exchange chromatography material is SP Sepharose® Fast Flow.

The terms "protein" and "polypeptide" refer to a polymer of amino acid residues with no limitation with respect to the minimum length of the polymer. Preferably, the protein or polypeptide has at least amino acids. The definition includes both full-length proteins and fragments thereof, as well as modifications thereof (e.g., glycosylation, phosphorylation, deletions, additions and substitutions). Preferably, the protein or polypeptide is an antibody.

The term "antibody" herein is used in the broadest sense and encompasses various antibody structures, including but not limited to monoclonal antibodies, polyclonal antibodies, multi-specific antibodies (e.g., bispecific antibodies), antibody-fusion proteins and antibody fragments so long as they exhibit the desired antigen-binding activity. The term also includes molecules like fusion proteins, in which further effector moieties are bound to the antibody. Also, Antibody Drug Conjugates (ADCs) are included.

As used herein, the term "effector moiety" refers to a polypeptide, e.g., a protein or glycoprotein, that influences cellular activity, for example, through signal transduction or other cellular pathways. Accordingly, the effector moiety of the invention may be associated with receptor-mediated signaling that transmits a signal from outside the cell membrane to modulate a response in a cell bearing one or more receptors for the effector moiety. An effector moiety may also elicit a cytotoxic response in cells bearing one or more receptors for the effector moiety. An effector moiety may also elicit a proliferative response in cells bearing one or more receptors for the effector moiety. An effector moiety can elicit differentiation in cells bearing receptors for the effector moiety. An effector moiety may also alter expression (i.e. upregulate or downregulate) of an endogenous cellular protein in cells bearing receptors for the effector moiety. Non-limiting examples of effector moieties include cytokines, growth factors, hormones, enzymes, substrates, and cofactors.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g. scFv); and multi-specific antibodies formed from antibody fragments (e.g. multi-specific Fabs). A Fab fragment is an antibody fragment obtained by a papain digestion of a (full length/complete) antibody.

"Multi-specific antibodies" are antibodies which have two different or more antigen-binding specificities. "Bispecific antibodies" are antibodies which have two different antigen-binding specificities. The term "bispecific" antibody as used herein denotes an antibody that has at least two binding sites each of which bind to different epitopes.

The terms "host cell", "host cell line", and "host cell culture" are used interchangeably and refer to cells into which exogenous nucleic acid has been introduced, including the progeny of such cells. Host cells include "transformants" and "transformed cells" which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. The term "cell" includes cells which are used for the expression of nucleic acids. Host cells may be a CHO cell (e.g. CHO K1, CHO DG44), or a BHK cell, or a NS0 cell, or a SP2/0 cell, or a HEK 293 cell, or a HEK 293 EBNA cell, or a PER.C6® cell, or a COS cell. As used herein, the expression "cell" includes the subject cell and its progeny.

In a further embodiment the protein is a recombinant protein. In a further embodiment the protein is an antibody. In a further embodiment the protein is a monoclonal antibody. In a further embodiment the protein is a multispecific antibody, a bispecific antibody or a monospecific antibody. In a further embodiment the protein is a humanized Type II anti-CD20 antibody. In a further embodiment the protein is a humanized Type II anti-CD20 antibody comprising (a) a heavy chain variable region of SEQ ID NO: 01, and (b) a light chain variable region of SEQ ID NO: 02. In a further embodiment the protein is obinutuzumab.

It has been found that the degree of purification is particularly pronounced if the solution comprising the polypeptide/protein is incubated/conditioned with a polysorbate/polysorbate-like surfactant before the chromatography and the solution comprising the polypeptide/protein is also washed with a solution comprising a polysorbate-like surfactant during the chromatography.

The term "about" denotes a range of +/- 20 % of the thereafter following numerical value. In one embodiment the term about denotes a range of +/- 10 % of the thereafter following numerical value. In one embodiment the term about denotes a range of +/- 5 % of the thereafter following numerical value.

To a person skilled in the art procedures and methods are well known to convert an amino acid sequence, e.g. of a polypeptide, into a corresponding nucleic acid sequence encoding this amino acid sequence. Therefore, a nucleic acid is characterized by its nucleic acid sequence consisting of individual nucleotides and likewise by the amino acid sequence of a polypeptide encoded thereby.

### Specific embodiments of the invention

1. A method for purifying a protein/polypeptide from a sample comprising the protein/polypeptide and at least one impurity with hydrolytic activity, comprising the following steps:
   i)
      a) applying the sample comprising the protein/polypeptide and at least one impurity with hydrolytic activity to a chromatography material,
      b) applying a (washing) solution which comprises a polysorbate/a polysorbate-like non-ionic surfactant to the chromatography material, and
      c) recovering the protein/polypeptide from the chromatography material, or
   ii)
      a) adding a polysorbate to the sample comprising the protein/polypeptide and at least one impurity with hydrolytic activity,
      b) applying the mixture of step a) to a chromatography material, and
      c) recovering the protein/polypeptide from the chromatography material, or
   iii)
      a) adding a polysorbate to the sample comprising the protein/polypeptide and at least one impurity with hydrolytic activity,
      b) applying the mixture of step a) to a chromatography material,
      c) applying a (washing) solution which comprises a polysorbate to the chromatography material, and
      d) recovering the protein/polypeptide from the chromatography material.
2. A method for purifying a protein/polypeptide from a sample comprising the protein/polypeptide and at least one impurity with hydrolytic activity, comprising the following steps:
   i)
      a) adding a polysorbate to the sample comprising the protein/polypeptide and at least one impurity with hydrolytic activity,
      b) applying the mixture of step a) to a chromatography material, and
      c) recovering the protein/polypeptide from the chromatography material, or
   ii)
      a) adding a polysorbate to the sample comprising the protein/polypeptide and at least one impurity with hydrolytic activity,
      b) applying the mixture of step a) to a chromatography material,
      c) applying a (washing) solution which comprises a polysorbate to the chromatography material, and
      d) recovering the protein/polypeptide from the chromatography material.
3. A method for purifying a protein/polypeptide from a sample comprising the protein/polypeptide and at least one impurity with hydrolytic activity, comprising the following steps:
   i)
      a) applying the sample comprising the protein/polypeptide and at least one impurity with hydrolytic activity to a chromatography material,
      b) applying a (washing) solution which comprises a polysorbate to the chromatography material, and
      c) recovering the protein/polypeptide from the chromatography material, or
   ii)
      a) adding a polysorbate to the sample comprising the protein/polypeptide and at least one impurity with hydrolytic activity,
      b) applying the mixture of step a) to a chromatography material,
      c) applying a (washing) solution which comprises a polysorbate to the chromatography material, and
      d) recovering the protein/polypeptide from the chromatography material.
4. A method for purifying a protein/polypeptide from a sample comprising the protein/polypeptide and at least one impurity with hydrolytic activity, comprising the following steps:
   a) applying the sample comprising the protein/polypeptide and at least one impurity with hydrolytic activity to a chromatography material,
   b) applying a (washing) solution which comprises a polysorbate to the chromatography material, and
   c) recovering the protein/polypeptide from the chromatography material.
5. A method for purifying a protein/polypeptide from a sample comprising the protein/polypeptide and at least one impurity with hydrolytic activity, comprising the following steps:
   a) adding a polysorbate to the sample comprising the protein/polypeptide and at least one impurity with hydrolytic activity,
   b) applying the mixture of step a) to a chromatography material, and
   c) recovering the protein from the chromatography material.
6. A method for purifying a protein/polypeptide from a sample comprising the protein/polypeptide and at least one impurity with hydrolytic activity, comprising the following steps:
   a) adding a polysorbate to the sample comprising the protein/polypeptide and at least one impurity with hydrolytic activity,
   b) applying the mixture of step a) to a chromatography material,
   c) applying a (washing) solution which comprises a polysorbate to the chromatography material, and
   d) recovering the protein/polypeptide from the chromatography material.
7. A method for reducing the hydrolytic activity (increasing polysorbate degradation stability) in a sample comprising a protein/polypeptide and at least one impurity with hydrolytic activity, comprising the following steps:
   i)
      a) applying the sample comprising the protein/polypeptide and at least one impurity with hydrolytic activity to a chromatography material,
      b) applying a (washing) solution which comprises a polysorbate to the chromatography material, and
      c) recovering the protein/polypeptide from the chromatography material, or
   ii)
      a) adding a polysorbate to the sample comprising the protein/polypeptide and at least one impurity with hydrolytic activity,
      b) applying the mixture of step a) to a chromatography material, and
      c) recovering the protein/polypeptide from the chromatography material, or
   iii)
      a) adding a polysorbate to the sample comprising the protein/polypeptide and at least one impurity with hydrolytic activity,
      b) applying the mixture of step a) to a chromatography material,
      c) applying a (washing) solution which comprises a polysorbate to the chromatography material, and
      d) recovering the protein/polypeptide from the chromatography material.
8. The method according to any one of embodiments 1 to 7, wherein the impurity (with hydrolytic activity) is an enzyme.
9. The method according to any one of embodiments 1 to 8, wherein the impurity is a hydrolase.
10. The method according to any one of embodiments 1 to 9, wherein the impurity is an esterase.
11. The method according to any one of embodiments 1 to 10, wherein the impurity is a lipase.
12. The method according to any one of embodiments 1 to 11, wherein the polysorbate is selected from the group comprising polysorbate 20, polysorbate 40, polysorbate 60, polysorbate 61, polysorbate 65, polysorbate 80, polysorbate 81, polysorbate 85 or polysorbate 120.
13. The method according to any one of embodiments 1 to 12, wherein the polysorbate is selected from the group comprising polysorbate 20 or polysorbate 80.
14. The method according to any one of embodiments 1 to 13, wherein the polysorbate is polysorbate 20.
15. The method according to any one of embodiments 1 to 13, wherein the polysorbate is polysorbate 80.
16. The method according to any one of embodiments 1 to 15, wherein the polysorbate is added to a final concentration of at least about 0.001% (w/v) and/or the washing solution comprising a polysorbate has a final concentration of at least about 0.001% (w/v).
17. The method according to any one of embodiments 1 to 16, wherein the polysorbate is added to a final concentration of at least about 0.005% (w/v) and/or the washing solution comprising a polysorbate has a final concentration of at least about 0.005% (w/v).
18. The method according to any one of embodiments 1 to 17, wherein the polysorbate is added to a final concentration of at least about 0.007% (w/v) and/or the washing solution comprising a polysorbate has a final concentration of at least about 0.007% (w/v).
19. The method according to any one of embodiments 1 to 18, wherein the polysorbate is added to a final concentration of at least about 0.01% (w/v) and/or the washing solution comprising a polysorbate has a final concentration of at least about 0.01% (w/v).
20. The method according to any one of embodiments 1 to 19, wherein the polysorbate is added to a final concentration of at least about 0.04% (w/v) and/or the washing solution comprising a polysorbate has a final concentration of at least about 0.04% (w/v).
21. The method according to any one of embodiments 1 to 20, wherein the polysorbate is added to a final concentration of from about 0.001% (w/v) to about 10% (w/v) and/or the washing solution comprising a polysorbate has a final concentration of at least about 0.001% (w/v) to about 10% (w/v).
22. The method according to any one of embodiments 1 to 21, wherein the chromatography material is an affinity chromatography material, a cation exchange chromatography material, an anion exchange chromatography material, a hydrophobic interaction chromatography material or a mixed mode chromatography material.
23. The method according to any one of embodiments 1 to 22, wherein the chromatography material is an affinity chromatography material, a cation exchange chromatography material or an anion exchange chromatography material.
24. The method according to any one of embodiments 1 to 23, wherein the chromatography material is an affinity chromatography material or a cation exchange chromatography material.
25. The method according to any one of embodiments 1 to 24, wherein the chromatography material is an affinity chromatography material.
26. The method according to any one of embodiments 1 to 25, wherein the chromatography material is an affinity chromatography material selected from the group comprising protein A or protein G or protein L affinity chromatography, single chain Fv ligand affinity chromatography, metal chelate affinity chromatography or an affinity chromatography material with a camelid-derived single domain antibody fragment as ligand (FcXL).
27. The method according to any one of embodiments 1 to 26, wherein the chromatography material is an affinity chromatography material selected from Protein A chromatography or a chromatography material with a camelid-derived single domain antibody fragment as ligand.
28. The method according to any one of embodiments 1 to 27, wherein the affinity chromatography material is a material with a matrix of aldehyde-activated agarose and a CaptureSelect FcXL affinity ligand [being a camelid-derived single domain antibody fragment] which specifically binds to the CH3 domain of all human IgG subclasses, wherein the ligand is coupled to the matrix by aldehyde coupling via NH2 residues of the ligand (CaptureSelect™ FcXL) or a material with a matrix of cross-linked agarose and an alkali-stabilized Protein A-derived ligand (MabSelect™ SuRe™).
29. The method according to any one of embodiments 1 to 28, wherein the affinity chromatography material is a material with a matrix of aldehyde-activated agarose, an average particle size of 65 µm and a CaptureSelect™ FcXL affinity ligand [being a camelid-derived single domain antibody fragment] which specifically binds to the CH3 domain of all human IgG subclasses, wherein the ligand is coupled to the matrix by aldehyde coupling via NH2 residues of the ligand (CaptureSelect™ FcXL) or a material with a matrix of cross-linked agarose, an alkali-stabilized Protein A-derived ligand and an average particle size of 85 µm (MabSelect™ SuRe™).
30. The method according to any one of embodiments 1 to 29, wherein the affinity chromatography material is CaptureSelect™ FcXL or MabSelect™ SuRe™.
31. The method according to any one of embodiments 1 to 24, wherein the chromatography material is a cation exchange chromatography material.
32. The method according to any one of embodiments 1 to 24 and 31, wherein the cation exchange chromatography material is a material with a matrix of cross-linked agarose and a sulphopropyl as ligand (SP Sepharose® Fast Flow).
33. The method according to any one of embodiments 1 to 24 and 31 to 32, wherein the cation exchange chromatography material is a material with a matrix of cross-linked agarose (6%), with a particle size of 45 to 165 µm (average 90 µm) and a sulphopropyl as ligand (SP Sepharose® Fast Flow).
34. The method according to any one of embodiments 1 to 24 and 31 to 33, wherein the cation exchange chromatography material is SP Sepharose® Fast Flow.
35. The method according to any one of embodiments 1 to 34, wherein the protein/polypeptide is a recombinant protein/polypeptide.
36. The method according to any one of embodiments 1 to 35, wherein the protein is an antibody.
37. The method according to any one of embodiments 1 to 36, wherein the protein/polypeptide is a monoclonal antibody.
38. The method according to any one of embodiments 1 to 37, wherein the protein/polypeptide is a multispecific antibody, a bispecific antibody or a monospecific antibody.
39. The method according to any one of embodiments 1 to 38, wherein the protein/polypeptide is a humanized Type II anti-CD20 antibody.
40. The method according to any one of embodiments 1 to 39, wherein the protein/polypeptide is a humanized Type II anti-CD20 antibody comprising
   (a) a heavy chain variable region of SEQ ID NO: 01, and
   (b) a light chain variable region of SEQ ID NO: 02.
41. The method according to any one of embodiments 1 to 40, wherein the protein/polypeptide is obinutuzumab.
42. Use of a polysorbate for reducing the hydrolytic activity (increasing polysorbate degradation stability) in a sample comprising a protein,
   wherein the sample comprising a protein is
   i) supplemented with a polysorbate before the application of the sample comprising the protein to a chromatography material, and/or
   ii) washed with a solution comprising a polysorbate after the application of the sample comprising the protein to a chromatography material.
43. The use according to embodiment 42, wherein the polysorbate is selected from the group polysorbate 20, polysorbate 40, polysorbate 60, polysorbate 61, polysorbate 65, polysorbate 80, polysorbate 81, polysorbate 85 or polysorbate 120.
44. The use according to any one of embodiments 42 to 43, wherein the polysorbate is selected from the group comprising polysorbate 20 or polysorbate 80.
45. The use according to any one of embodiments 42 to 44, wherein the polysorbate is polysorbate 20.
46. The use according to any one of embodiments 42 to 44, wherein the polysorbate is polysorbate 80.
47. The use according to any one of embodiments 42 to 46, wherein the polysorbate is added to a final concentration of at least about 0.001% (w/v).
48. The use according to any one of embodiments 42 to 47, wherein the polysorbate is added to a final concentration of at least about 0.005% (w/v).
49. The use according to any one of embodiments 42 to 48, wherein the polysorbate is added to a final concentration of at least about 0.007% (w/v).
50. The use according to any one of embodiments 42 to 49, wherein the polysorbate is added to a final concentration of at least about 0.01% (w/v).
51. The use according to any one of embodiments 42 to 50, wherein the polysorbate is added to a final concentration of at least about 0.04% (w/v).
52. The use according to any one of embodiments 42 to 51, wherein the polysorbate is added to a final concentration of from about 0.001% (w/v) to about 1% (w/v).
53. The use according to any one of embodiments 42 to 52, wherein the chromatography material is an affinity chromatography material, a cation exchange chromatography material, an anion exchange chromatography material, a hydrophobic interaction chromatography material or a mixed mode chromatography material.
54. The use according to any one of embodiments 42 to 53, wherein the chromatography material is an affinity chromatography material, a cation exchange chromatography material or an anion exchange chromatography material.
55. The use according to any one of embodiments 42 to 54, wherein the chromatography material is an affinity chromatography material or a cation exchange chromatography material.
56. The use according to any one of embodiments 42 to 55, wherein the chromatography material is an affinity chromatography material.
57. The use according to any one of embodiments 42 to 56, wherein the chromatography material is an affinity chromatography material selected from the group comprising protein A or protein G or protein L affinity chromatography, single chain Fv ligand affinity chromatography, metal chelate affinity chromatography or an affinity chromatography material with a camelid-derived single domain antibody fragments as ligand (FcXL).
58. The use according to any one of embodiments 42 to 57, wherein the chromatography material is an affinity chromatography material selected from Protein A chromatography or a chromatography material with a camelid-derived single domain antibody fragments as ligand.
59. The use according to any one of embodiments 42 to 58, wherein the affinity chromatography material is a material with a matrix of aldehyde-activated agarose and a CaptureSelect FcXL affinity ligand [being a camelid-derived single domain antibody fragment] which specifically binds to the CH3 domain of all human IgG subclasses, wherein the ligand is coupled to the matrix by aldehyde coupling via NH2 residues of the ligand (CaptureSelect™ FcXL) or a material with a matrix of cross-linked agarose and an alkali-stabilized Protein A-derived ligand (MabSelect™ SuRe™).
60. The use according to any one of embodiments 42 to 59, wherein the affinity chromatography material is a material with a matrix of aldehyde-activated agarose, an average particle size of 65 µm and a CaptureSelect FcXL affinity ligand [being a camelid-derived single domain antibody fragment] which specifically binds to the CH3 domain of all human IgG subclasses, wherein the ligand is coupled to the matrix by aldehyde coupling via NH2 residues of the ligand (CaptureSelect™ FcXL) or a material with a matrix of cross-linked agarose, an alkali-stabilized Protein A-derived ligand and an average particle size of 85 µm (MabSelect™ SuRe™).
61. The use according to any one of embodiments 42 to 55, wherein the affinity chromatography material is CaptureSelect™ FcXL or MabSelect™ SuRe™.
62. The use according to any one of embodiments 42 to 55, wherein the chromatography material is a cation exchange chromatography material.
63. The use according to any one of embodiments 42 to 55 and 62, wherein the cation exchange chromatography material is a material with a matrix of cross-linked agarose and a sulphopropyl as ligand (SP Sepharose® Fast Flow).
64. The use according to any one of embodiments 42 to 55 and 62 to 63, wherein the cation exchange chromatography material is a material with a matrix of cross-linked agarose (6%), with a particle size of 45 to 165 µm (average 90 µm) and a sulphopropyl as ligand (SP Sepharose® Fast Flow).
65. The use according to any one of embodiments 42 to 55 and 62 to 64, wherein the cation exchange chromatography material is SP Sepharose® Fast Flow.
66. The use according to any one of embodiments 42 to 65, wherein the protein is a recombinant protein.
67. The use according to any one of embodiments 42 to 66, wherein the protein is an antibody.
68. The use according to any one of embodiments 42 to 67, wherein the protein is a monoclonal antibody.
69. The use according to any one of embodiments 42 to 68, wherein the protein is a multispecific antibody, a bispecific antibody or a monospecific antibody.
70. The use according to any one of embodiments 42 to 69, wherein the protein is a humanized Type II anti-CD20 antibody.
71. The use according to any one of embodiments 42 to 70, wherein the protein is a humanized Type II anti-CD20 antibody comprising
   (a) a heavy chain variable region of SEQ ID NO: 01, and
   (b) a light chain variable region of SEQ ID NO: 02.
72. The use according to any one of embodiments 42 to 71, wherein the protein is obinutuzumab.
73. Liquid antibody preparation (comprising a polysorbate) with reduced hydrolytic activity (increased polysorbate degradation stability) obtained by a method according to any one of embodiments 1 to 41 or 77 to 107.
74. The liquid antibody preparation according to embodiment 73, wherein the antibody is a humanized Type II anti-CD20 antibody.
75. The liquid antibody preparation according to any one of embodiments 73 to 74, wherein the antibody is a humanized Type II anti-CD20 antibody comprising
   (a) a heavy chain variable region of SEQ ID NO: 01, and
   (b) a light chain variable region of SEQ ID NO: 02.
76. The liquid antibody preparation according to any one of embodiments 73 to 75, wherein the antibody is obinutuzumab.
77. A method for reducing the amount of hydrolytic enzymes in a sample comprising a protein and hydrolytic enzymes, comprising the following steps:
   a) adding a polysorbate to the sample comprising a protein and hydrolytic enzymes before the application of the sample comprising the protein and hydrolytic enzymes to a chromatography material, and/or
   b) washing with a solution comprising a polysorbate after the application of the sample comprising the protein and hydrolytic enzymes to a chromatography material.
78. The method according to embodiment 77, wherein the polysorbate is selected from the group comprising polysorbate 20, polysorbate 40, polysorbate 60, polysorbate 61, polysorbate 65, polysorbate 80, polysorbate 81, polysorbate 85 or polysorbate 120.
79. The method according to any one of embodiments 77 to 78, wherein the polysorbate is selected from the group comprising polysorbate 20 or polysorbate 80.
80. The method according to any one of embodiments 77 to 79, wherein the polysorbate is polysorbate 20.
81. The method according to any one of embodiments 77 to 79, wherein the polysorbate is polysorbate 80.
82. The method according to any one of embodiments 77 to 81, wherein the polysorbate is added to a final concentration of at least about 0.001% (w/v) and/or the washing solution comprising a polysorbate has a final concentration of at least about 0.001% (w/v).
83. The method according to any one of embodiments 77 to 82, wherein the polysorbate is added to a final concentration of at least about 0.005% (w/v) and/or the washing solution comprising a polysorbate has a final concentration of at least about 0.005% (w/v).
84. The method according to any one of embodiments 77 to 83, wherein the polysorbate is added to a final concentration of at least about 0.007% (w/v) and/or the washing solution comprising a polysorbate has a final concentration of at least about 0.007% (w/v).
85. The method according to any one of embodiments 77 to 84, wherein the polysorbate is added to a final concentration of at least about 0.01% (w/v) and/or the washing solution comprising a polysorbate has a final concentration of at least about 0.01% (w/v).
86. The method according to any one of embodiments 77 to 85, wherein the polysorbate is added to a final concentration of at least about 0.04% (w/v) and/or the washing solution comprising a polysorbate has a final concentration of at least about 0.04% (w/v).
87. The method according to any one of embodiments 77 to 86, wherein the polysorbate is added to a final concentration of from about 0.001% (w/v) to about 1% (w/v) and/or the washing solution comprising a polysorbate has a final concentration of from about 0.001% (w/v) to about 1% (w/v).
88. The method according to any one of embodiments 77 to 87, wherein the chromatography material is an affinity chromatography material, a cation exchange chromatography material, an anion exchange chromatography material, a hydrophobic interaction chromatography material or a mixed mode chromatography material.
89. The method according to any one of embodiments 77 to 88, wherein the chromatography material is an affinity chromatography material, a cation exchange chromatography material or an anion exchange chromatography material.
90. The method according to any one of embodiments 77 to 89, wherein the chromatography material is an affinity chromatography material or a cation exchange chromatography material.
91. The method according to any one of embodiments 77 to 90, wherein the chromatography material is an affinity chromatography material.
92. The method according to any one of embodiments 77 to 91, wherein the chromatography material is an affinity chromatography material selected from the group comprising protein A or protein G or protein L affinity chromatography, single chain Fv ligand affinity chromatography, metal chelate affinity chromatography or an affinity chromatography material with a camelid-derived single domain antibody fragments as ligand (FcXL).
93. The method according to any one of embodiments 77 to 92, wherein the chromatography material is an affinity chromatography material selected from Protein A chromatography or a chromatography material with a camelid-derived single domain antibody fragments as ligand.
94. The method according to any one of embodiments 77 to 93, wherein the affinity chromatography material is a material with a matrix of aldehyde-activated agarose and a CaptureSelect FcXL affinity ligand [being a camelid-derived single domain antibody fragment] which specifically binds to the CH3 domain of all human IgG subclasses, wherein the ligand is coupled to the matrix by aldehyde coupling via NH2 residues of the ligand (CaptureSelect™ FcXL) or a material with a matrix of cross-linked agarose and an alkali-stabilized Protein A-derived ligand (MabSelect™ SuRe™).
95. The method according to any one of embodiments 77 to 94, wherein the affinity chromatography material is a material with a matrix of aldehyde-activated agarose, an average particle size of 65 µm and a CaptureSelect FcXL affinity ligand [being a camelid-derived single domain antibody fragment] which specifically binds to the CH3 domain of all human IgG subclasses, wherein the ligand is coupled to the matrix by aldehyde coupling via NH2 residues of the ligand (CaptureSelect™ FcXL) or a material with a matrix of cross-linked agarose, an alkali-stabilized Protein A-derived ligand and an average particle size of 85 µm (MabSelect™ SuRe™).
96. The method according to any one of embodiments 77 to 95, wherein the affinity chromatography material is CaptureSelect™ FcXL or MabSelect™ SuRe™.
97. The method according to any one of embodiments 77 to 90, wherein the chromatography material is a cation exchange chromatography material.
98. The method according to any one of embodiments 77 to 90 and 97, wherein the cation exchange chromatography material is a material with a matrix of cross-linked agarose and a sulphopropyl as ligand (SP Sepharose® Fast Flow).
99. The method according to any one of embodiments 77 to 90 and 97 to 98, wherein the cation exchange chromatography material is a material with a matrix of cross-linked agarose (6%), with a particle size of 45 to 165 µm (average 90 µm) and a sulphopropyl as ligand (SP Sepharose® Fast Flow).
100. The method according to any one of embodiments 77 to 90 and 97 to 99, wherein the cation exchange chromatography material is SP Sepharose® Fast Flow.
101. The method according to any one of embodiments 77 to 100, wherein the protein is a recombinant protein.
102. The method according to any one of embodiments 77 to 101, wherein the protein is an antibody.
103. The method according to any one of embodiments 77 to 102, wherein the protein is a monoclonal antibody.
104. The method according to any one of embodiments 77 to 103, wherein the protein is a multispecific antibody, a bispecific antibody or a monospecific antibody.
105. The method according to any one of embodiments 77 to 104, wherein the protein is a humanized Type II anti-CD20 antibody.
106. The method according to any one of embodiments 77 to 105, wherein the protein is a humanized Type II anti-CD20 antibody comprising
   (a) a heavy chain variable region of SEQ ID NO: 01, and
   (b) a light chain variable region of SEQ ID NO: 02.
107. The method according to any one of embodiments 77 to 107, wherein the protein is obinutuzumab.
108. A method for producing a (recombinant) protein comprising the following steps:
   a) cultivating a mammalian cell comprising a nucleic acid encoding an (recombinant) protein,
   b) recovering the (recombinant) protein from the cell or the cultivation medium, and
   c) purifying the (recombinant) protein with a method according to any one of embodiments 1 to 41,
   and thereby producing the (recombinant) protein.
109. A liquid antibody preparation with increased stability obtained by a method according to any one of embodiments 1 to 41 or 77 to 107 or 111 to 114.
110. A liquid antibody preparation with reduced particle formation (formed by free fatty acid assemblies) obtained by a method according to any one of embodiments 1 to 41 or 77 to 107 or 111 to 114.
111. The method according to any one of embodiments 1 to 41 or 77 to 107, wherein the polysorbate is added to a final concentration of from about 0.001% (w/v) to about 5% (w/v) and/or the washing solution comprising a polysorbate has a final concentration of at least about 0.001% (w/v) to about 5% (w/v).
112. The method according to any one of embodiments 1 to 41 or 77 to 107 or 111, wherein the polysorbate is added to a final concentration of from about 0.001% (w/v) to about 3% (w/v) and/or the washing solution comprising a polysorbate has a final concentration of at least about 0.001% (w/v) to about 3% (w/v).
113. The method according to any one of embodiments 1 to 41 or 77 to 107 or 111 to 112, wherein the polysorbate is added to a final concentration of from about 0.001% (w/v) to about 2% (w/v) and/or the washing solution comprising a polysorbate has a final concentration of at least about 0.001% (w/v) to about 2% (w/v).
114. The method according to any one of embodiments 1 to 41 or 77 to 107 or 111 to 113, wherein the polysorbate is added to a final concentration of from about 0.001% (w/v) to about 1% (w/v) and/or the washing solution comprising a polysorbate has a final concentration of at least about 0.001% (w/v) to about 1% (w/v).
115. The method according to any one of embodiments 1 to 41 or 77 to 107 or 111 to 114, wherein the chromatography material is in a column.
116. The method according to any one of embodiments 1 to 41 or 77 to 107 or 111 to 114, wherein the chromatography material is suspended in the solution/sample.

### References

[1] Kerwin, Bruce A. (2008): Polysorbates 20 and 80 used in the formulation of protein biotherapeutics. Structure and degradation pathways. In Journal of pharmaceutical sciences 97 (8), pp. 2924-2935. DOI: 10.1002/jps.21190.
[2] Wang, Wei (2005): Protein aggregation and its inhibition in biopharmaceutics. In International journal of pharmaceutics 289 (1-2), pp. 1-30. DOI: 10.1016/j.ijpharm.2004.11.014.
[3] Jones LS, Bam NB, Randolph TW. Surfactant-stabilizedprotein formulations: a review of protein-surfactant interactions and novel analytical methodologies
[4] Kreilgaard, L.; Jones, L. S.; Randolph, T. W.; Frokjaer, S.; Flink, J. M.; Manning, M. C.; Carpenter, J. F. (1998): Effect of Tween 20 on freeze-thawing- and agitation-induced aggregation of recombinant human factor XIII. In Journal of pharmaceutical sciences 87 (12), pp. 1597-1603.
[5] Hillgren, Anna; Lindgren, Jan; Alden, Maggie (2002): Protection mechanism of Tween 80 during freeze-thawing of a model protein, LDH. In International journal of pharmaceutics 237 (1-2), pp. 57-69.
[6] Chi, Eva Y.; Krishnan, Sampathkumar; Randolph, Theodore W.; Carpenter, John F. (2003): Physical stability of proteins in aqueous solution. Mechanism and driving forces in nonnative protein aggregation. In Pharmaceutical research 20 (9), pp. 1325-1336.
[7] Maa, Y. F.; Hsu, C. C. (1997): Protein denaturation by combined effect of shear and air-liquid interface. In Biotechnology and bioengineering 54 (6), pp. 503-512. DOI: 10.1002/(SICI)1097-0290(19970620)54:6<503::AID-BIT1>3.0.CO;2-N.
[8] Randolph TW, Jones LS. Surfactant-protein interactions. Rational design of stable protein formulations
[9] Bam, N. B.; Cleland, J. L.; Yang, J.; Manning, M. C.; Carpenter, J. F.; Kelley, R. F.; Randolph, T. W. (1998): Tween protects recombinant human growth hormone against agitation-induced damage via hydrophobic interactions. In Journal of pharmaceutical sciences 87 (12), pp. 1554-1559.
[10] Ayorinde, F. O.; Gelain, S. V.; Johnson, J. H., JR; Wan, L. W. (2000): Analysis of some commercial polysorbate formulations using matrix-assisted laser desorption/ionization time-of-flight mass spectrometry. In Rapid communications in mass spectrometry: RCM 14 (22), pp. 2116-2124. DOI: 10.1002/1097-0231(20001130)14:22<2116: AID-RCM142>3.0.CO;2-1.
[11] Zhang, R.; Wang, Y.; Tan, L.; Zhang, H. Y.; Yang, M. (2012): Analysis of polysorbate 80 and its related compounds by RP-HPLC with ELSD and MS detection. In Journal of chromatographic science 50 (7), pp. 598-607. DOI: 10.1093/chromsci/bms035.
[12] Vu Dang, Hoang; Gray, Alexander I.; Watson, David; Bates, Catharine D.; Scholes, Peter; Eccleston, Gillian M. (2006): Composition analysis of two batches of polysorbate 60 using MS and NMR techniques. In Journal of pharmaceutical and biomedical analysis 40 (5), pp. 1155-1165. DOI: 10.1016/j.jpba.2005.10.007.
[13] Hewitt, Daniel; Alvarez, Melissa; Robinson, Kathryn; Ji, Junyan; Wang, Y. John; Kao, Yung-Hsiang; Zhang, Taylor (2011): Mixed-mode and reversed-phase liquid chromatography-tandem mass spectrometry methodologies to study composition and base hydrolysis of polysorbate 20 and 80. In Journal of chromatography. A 1218 (15), pp. 2138-2145. DOI: 10.1016/j.chroma.2010.09.057.
[14] Brandner, J. D. (1998): The composition of NF-defined emulsifiers. Sorbitan monolaurate, monopalmitate, monostearate, monooleate, polysorbate 20, polysorbate 40, polysorbate 60, and polysorbate 80. In Drug development and industrial pharmacy 24 (11), pp. 1049-1054. DOI: 10.3109/03639049809089948.
[15] EP monograph 01/2005:0426
[16] USP [9005-64-5]
[17] Kishore, Ravuri S. K.; Pappenberger, Astrid; Dauphin, Isabelle Bauer; Ross, Alfred; Buergi, Beatrice; Staempfli, Andreas; Mahler, Hanns-Christian (2011): Degradation of polysorbates 20 and 80. Studies on thermal autoxidation and hydrolysis. In Journal of pharmaceutical sciences 100 (2), pp. 721-731. DOI: 10.1002/jps.22290.
[18] Labrenz, Steven R. (2014): Ester hydrolysis of polysorbate 80 in mAb drug product. Evidence in support of the hypothesized risk after the observation of visible particulate in mAb formulations. In Journal of pharmaceutical sciences 103 (8), pp. 2268-2277. DOI: 10.1002/jps.24054.
[19] Tomlinson, Anthony; Demeule, Barthelemy; Lin, Baiwei; Yadav, Sandeep (2015): Polysorbate 20 Degradation in Biopharmaceutical Formulations. Quantification of Free Fatty Acids, Characterization of Particulates, and Insights into the Degradation Mechanism. In: Molecular pharmaceutics 12 (11), S. 3805-3815. DOI: 10.1021/acs.molpharmaceut.5b00311.
[20] Torosantucci, Riccardo; Schoneich, Christian; Jiskoot, Wim (2014): Oxidation of therapeutic proteins and peptides. Structural and biological consequences. In Pharmaceutical research 31 (3), pp. 541-553. DOI: 10.1007/s11095-013-1199-9
[21] Saggu, Miguel; Liu, Jun; Patel, Ankit (2015): Identification of Subvisible Particles in Biopharmaceutical Formulations Using Raman Spectroscopy Provides Insight into Polysorbate 20 Degradation Pathway. In Pharmaceutical research 32 (9), pp. 2877-2888. DOI: 10.1007/s11095-015-1670-x.
[22] Chiu, Josephine; Valente, Kristin N.; Levy, Nicholas E.; Min, Lie; Lenhoff, Abraham M.; Lee, Kelvin H. (2017): Knockout of a difficult-to-remove CHO host cell protein, lipoprotein lipase, for improved polysorbate stability in monoclonal antibody formulations. In Biotechnology and bioengineering 114 (5), pp. 1006-1015. DOI: 10.1002/bit.26237.
[23] Doneanu, Catalin E.; Xenopoulos, Alex; Fadgen, Keith; Murphy, Jim; Skilton, St John; Prentice, Holly et al. (2012): Analysis of host-cell proteins in biotherapeutic proteins by comprehensive online two-dimensional liquid chromatography/mass spectrometry. In mAbs 4 (1), pp. 24-44. DOI: 10.4161/mabs.4.1.18748.
[24] Dixit, Nitin; Salamat-Miller, Nazila; Salinas, Paul A.; Taylor, Katherine D.; Basu, Sujit K. (2016): Residual Host Cell Protein Promotes Polysorbate 20 Degradation in a Sulfatase Drug Product Leading to Free Fatty Acid Particles. In Journal of pharmaceutical sciences 105 (5), pp. 1657-1666. DOI: 10.1016/j.xphs.2016.02.029.

The following examples, sequences and figures are provided to aid the understanding of the present invention, the true scope of which is set forth in the appended claims. It is understood that modifications can be made in the procedures set forth without departing from the spirit of the invention.

### Description of the Sequence Listing

- **SEQ ID NO: 01**: Amino acid sequence of heavy chain variable region (VH) of humanized Type II anti-CD20 antibody (obinutuzumab).
- **SEQ ID NO: 02**: Amino acid sequence of light chain variable region (VL) of humanized Type II anti-CD20 antibody (obinutuzumab).

### Description of the Figures

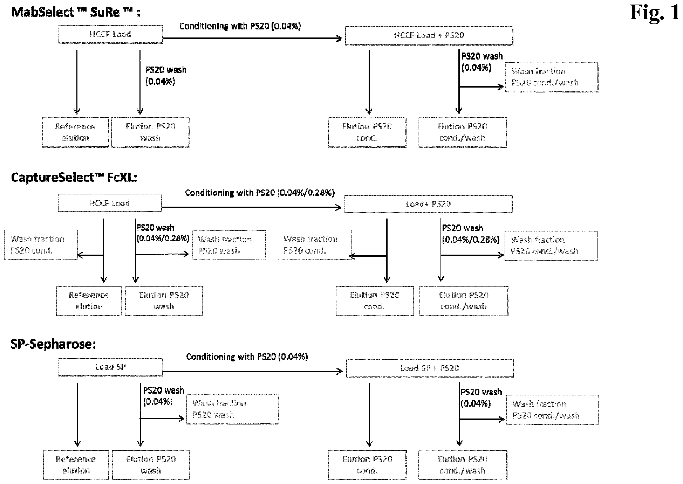

- **Figure 1**: Schematic overview of the applied exemplary downstream processing workflows including an optional pre-incubation step (conditioning of HCCF Load/ Load SP with polysorbate 20 (PS20)), washing of the bound mAb with a PS20-containing solution or a combination of both steps, respectively. The hydrolytic activity (measured as remaining PS20 content as a function of time) of the corresponding elution fractions (Elution PS 20 cond., Elution PS20 wash and PS20 cond./wash) was compared to the Reference Elution using standard chromatography conditions.

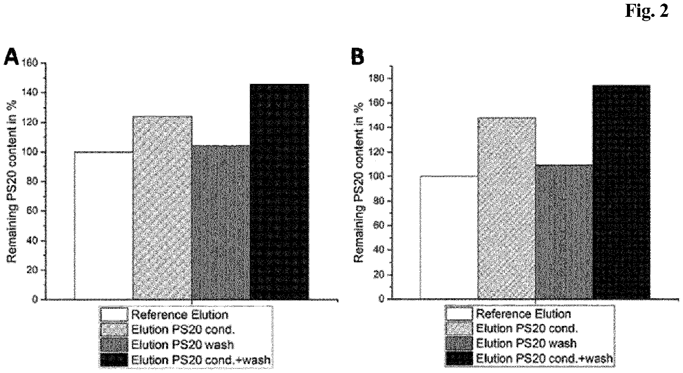

- **Figure 2**: Remaining PS20 content of the MabSelect SuRe elution fractions after an incubation time of 238h (A) and 317h (B) at an employed PS20 concentration for downstream processing of 0.04% (w/v) referred to the relative PS20 content of the corresponding Reference Elution. Higher remaining PS20 content translates to reduced hydrolytic activity.

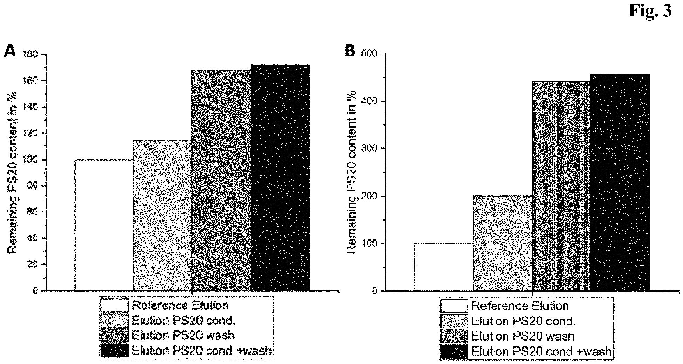

- **Figure 3**: Remaining PS20 content of the CaptureSelect FcXL elution fractions after an incubation time of 86h (A) and 163h (B) at an employed PS20 concentration for downstream processing of 0.04% (w/v) referred to the relative PS20 content of the corresponding Reference Elution. Higher remaining PS20 content translates to reduced hydrolytic activity.

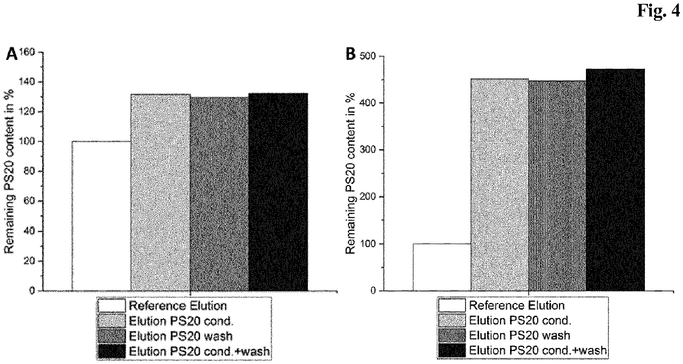

- **Figure 4**: Remaining PS20 content of the CaptureSelect™ FcXL elution fractions after an incubation time of 86h (A) and 163h (B) at an employed PS20 concentration for downstream processing of 0.28% (w/v) referred to the relative PS20 content of the corresponding Reference Elution. Higher remaining PS20 content translates to reduced hydrolytic activity.

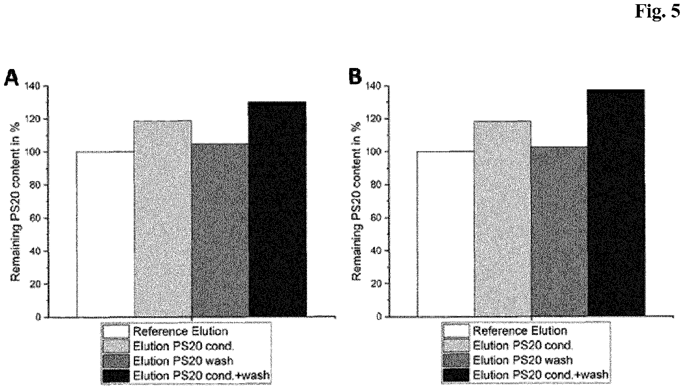

- **Figure 5**: Remaining PS20 content of the SP Sepharose Fast Flow elution fractions after an incubation time of 238h (A) and 317h (B) at an employed PS20 concentration for downstream processing of 0.04% (w/v) referred to the relative PS20 content of the corresponding Reference Elution. Higher remaining PS20 content translates to reduced hydrolytic activity.

### Examples

### General methods

### Determination of protein purity

Protein purity was determined by size-exclusion High Performance Liquid Chromatography (SE-HPLC) using a Dionex UltiMate 3000 HPLC system (Thermo Scientific). Protein separation was performed on a TSKGEL G3000SWXL 7.8X300 column (Tosoh Bioscience LLC) with a flow rate of 0.5 ml/min using 0.2 M K₂HPO₄/KH₂PO₄, 0.25 M KCl, pH 7.0 as running buffer.

### Determination of protein concentration

Protein concentrations were determined by UV spectroscopy using a Gary® 50 UV-Vis Spectrophotometer (Varian). Protein samples were diluted in their respective buffers and measured as duplicates. Concentrations were determined according to the following equation deriving from Lambert-Beer law: *c* = (*A*_{280 nm} - *A*_{320 nm})/ *ε* · *d·* F with *c* protein concentration [mg/ml], *A* absorbance, ε extinction coefficient [ml/(mg·cm)], *d* cell length [cm] and F dilution factor. The obinutuzumab-specific extinction coefficient is 1.49 ml/(mg·cm).

### Antibodies

The current invention is exemplified using exemplary antibodies such as an humanized Type II anti-CD20 antibody as described in WO 2005/044859 or SEQ ID NO: 01 to SEQ ID NO: 02.

### Purification overview

A schematic overview of the applied exemplary downstream processes is depicted in Figure 1.

### Example 1

### Purification of an anti-CD20 antibody in a Protein A chromatography (MabSelect SuRe)

### Load preparation:

Following steps were performed sequentially:
1) Untreated HCCF containing obinutuzumab (anti-CD20) was thawed and filtered using a 0.22 µm pore size filter unit (Merck Millipore).
2) HCCF was split into four identical portions. In case of PS20 conditioning, a final concentration of 0.04 % (w/v) PS20 was added to the HCCF. The conditioned solutions were homogenized by stirring (300 rpm, 10 min, RT) and incubated for 24 h at 4 °C without stirring. All portions were stored at - 20 °C.

### Chromatography:

Chromatographic protein purification was performed using a MabSelect™ SuRe™ column (Ø 1 cm, h = 25.5 cm, V = 20.3 ml) (GE Healthcare) on an AEKTA Explorer 100 system (GE Healthcare) and a constant flow rate of 440 cm/h during all steps. Run details are listed as follows:

| Run 1 (Reference): | | |
|---|---|---|
| Load density: | | 38.2 g/Lᵣₑₛᵢₙ |
| Chromatographic steps: | | |
| | 1. Equilibration | 25 mM Tris/HCl, 25 mM NaCl, pH 7.0 |
| | 2. Load HCCF | |
| | 3. Wash 1 | 25 mM Tris/HCl, 25 mM NaCl, pH 7.0 |
| | 4. Wash 2 | 0.7 M Tris/HCl, pH 7.2 |
| | 5. Wash 3 | 25 mM Tris/HCl, 25 mM NaCl, pH 7.0 |
| | 6. Elution | 30 mM acetic acid (Pool 0.25 AU - 0.25 AU (1 cm UV-cell)) |
| | | |

| Run 2 (conditioned HCCF containing 0.04 % (w/v) PS20): | | |
|---|---|---|
| Load density: | | 37.45 g/Lᵣₑₛᵢₙ |
| Chromatographic steps: | | |
| | 1. Equilibration | 25 mM Tris/HCl, 25 mM NaCl, pH 7.0 |
| | 2. Load HCCF | conditioned HCCF containing 0.04 % (w/v) PS20 |
| | 3. Wash 1 | 25 mM Tris/HCl, 25 mM NaCl, pH 7.0 |
| | 4. Wash 2 | 0.7 M Tris/HCl, pH 7.2 |
| | 5. Wash 3 | 25 mM Tris/HCl, 25 mM NaCl, pH 7.0 |
| | 6. Elution | 30 mM Acetic acid (Pool 0.25 AU - 0.25 AU (1 cm UV-cell)) |
| | | |

| Run 3 (add. wash-strip 0.04 % (w/v) PS20): | | |
|---|---|---|
| Load densitiy: | | 38.2 g/Lᵣₑₛᵢₙ |
| Chromatographic steps: | | |
| | 1. Equilibration | 25 mM Tris/HCl, 25 mM NaCl, pH 7.0 |
| | 2. Load HCCF | |
| | 3. Wash 1 | 25 mM Tris/HCl, 25 mM NaCl, pH 7.0 |
| | 4. Wash 2 | 0.04 % (w/v) PS20 (5 CV in total: 4 CV, 30 min hold, 1 CV) |
| | 5. Wash 3 | 0,7 M Tris/HCl, pH 7.2 |
| | 6. Wash 4 | 25 mM Tris/HCl, 25 mM NaCl, pH 7.0 |
| | 7. Elution | 30 mM Acetic acid (Pool 0.25 AU - 0.25 AU (1 cm UV-cell)) |
| | | |

| Run 4 (conditioned HCCF containing 0.04 % (w/v) PS20 + add. wash-strip 0.04 % (w/v) PS20): | | |
|---|---|---|
| Load density: | | 37.45 g/Lᵣₑₛᵢₙ |
| Chromatographic steps: | | |
| | 1. Equilibration | 25 mM Tris/HCl, 25 mM NaCl, pH 7.0 |
| | 2. Load HCCF | conditioned HCCF containing 0.04 % (w/v) PS20 |
| | 3. Wash 1 | 25 mM Tris/HCl, 25 mM NaCl, pH 7.0 |
| | 4. Wash 2: | 0.04 % (w/v) PS20 (5 CV in total: 4 CV, 30 min hold, 1 CV) |
| | 5. Wash 3 | 0.7 M Tris/HCl, pH 7.2 |
| | 6. Wash 4 | 25 mM Tris/HCl, 25 mM NaCl, pH 7.0 |
| | 7. Elution 30 | mM Acetic acid (Pool 0.25 AU - 0.25 AU (1 cm UV-cell)) |

### MSS run pool summary:

The MabSelect SuRe® eluate (containing obinutuzumab) was adjusted to a pH value of 6,0 ± 0,2 with 1.5 M Tris-amino and consecutively filtered using a 0.45 µm and a 0.22 µm pore size Minisart® filter unit (Sartorius Stedim). Filtered eluate was frozen at -20 °C and served as starting material for subsequent analytics.

| **Run** | **PS20 conditioning in HCCF** | **additional PS20 wash-strip** | **Yield Eluate** | **SE-HPLC Main Peak** |
|---|---|---|---|---|
| | **c(PS20) [% (w/v)]** | **c(PS20) [% (w/v)]** | **[%]** | **[%]** |
| 1 | - | - | 90.99 | 99.01 |
| 2 | 0.04 | - | 90.89 | 99.28 |
| 3 | - | 0.04 | 91.17 | 99.16 |
| 4 | 0.04 | 0.04 | 95.22 | 98.93 |

### Example 2

### Purification of an anti-CD20 antibody in an anti-IgG Fc chromatography/ chromatography using a material with a camelid-derived single domain antibody fragment as ligand (CaptureSelect™ FcXL)

### Load preparation:

Following steps were performed sequentially:
1) Untreated HCCF containing obinutuzumab (anti-CD20) was thawed and filtered using a 0.22 µm pore size filter unit (Merck Millipore).
2) HCCF was split into seven identical portions. In case of PS20 conditioning, a final concentration of either 0.04 % (w/v) PS20 or 0.28 % (w/v) was added to the HCCF. The conditioned solutions were homogenized by stirring (300 rpm, 10 min, RT) and incubated for 24 h at 4 °C without stirring. All portions were stored at -20 °C.

### Chromatography:

Chromatographic protein purification was performed using a CaptureSelect FcXL column (Ø 1 cm, h = 20.5 cm, V = 16.1 ml) (Thermo Fisher) on an AEKTA AVANT 150 system (GE Healthcare) and a constant flow rate of 200 cm/h during all steps. Run details are listed as follows:

| Run 1 (Reference): | | |
|---|---|---|
| Load density: | | 19.81 g/Lᵣₑₛᵢₙ |
| Chromatographic steps: | | |
| | 1. Equilibration | 25 mM Tris/HCl, 25 mM NaCl, pH 7.0 |
| | 2. Load HCCF | |
| | 3. Wash 1 | 25 mM Tris/HCl, 25 mM NaCl, pH 7.0 |
| | 4. Wash 2 | purified water (type II) |
| | 5. Wash 3 | 25 mM Tris/HCl, 25 mM NaCl, pH 7.0 |
| | 6. Elution | 30 mM Acetic acid (Pool 2.5 AU - 2.5 AU (1 cm UV-cell)) |
| | | |

| Run 2 (conditioned HCCF containing 0.04 % (w/v) PS20): | | |
|---|---|---|
| Load density: | | 19.75 g/Lᵣₑₛᵢₙ |
| Chromatographic steps: | | |
| | 1. Equilibration | 25 mM Tris/HCl, 25 mM NaCl, pH 7.0 |
| | 2. Load HCCF | conditioned HCCF containing 0.04 % (w/v) PS20 |
| | 3. Wash 1 | 25 mM Tris/HCl, 25 mM NaCl, pH 7.0 |
| | 4. Wash 2 | purified water (type II) |
| | 5. Wash 3 | 25 mM Tris/HCl, 25 mM NaCl, pH 7.0 |
| | 6.Elution | 30 mM Acetic acid (Pool 2.5 AU - 2.5 AU (1 cm UV-cell)) |
| | | |

| Run 3 (add. wash-strip 0.04 % (w/v) PS20): | | |
|---|---|---|
| Load density: | | 19.81 g/Lᵣₑₛᵢₙ |
| Chromatographic steps: | | |
| | 1. Equilibration | 25 mM Tris/HCl, 25 mM NaCl, pH 7.0 |
| | 2. Load HCCF | |
| | 3. Wash 1 | 25 mM Tris/HCl, 25 mM NaCl, pH 7.0 |
| | 4. Wash 2 | 0.04 % (w/v) PS20 (5 CV in total: 4 CV, 30 min hold, 1 CV) |
| | 5. Wash 3 | purified water (type II) |
| | 6. Wash 4 | 25 mM Tris/HCl, 25 mM NaCl, pH 7.0 |
| | 7. Elution | 30 mM Acetic acid (Pool 2.5 AU - 2.5 AU (1 cm UV-cell)) |
| | | |

| Run 4 (conditioned HCCF containing 0.04 % (w/v) PS20 + add. wash-strip 0.04 % (w/v) PS20) | | |
|---|---|---|
| Load densitiy: | | 19.77 g/Lᵣₑₛᵢₙ |
| Chromatographic steps: | | |
| | 1. Equilibration | 25 mM Tris/HCl, 25 mM NaCl, pH 7.0 |
| | 2. Load HCCF | conditioned HCCF containing 0.04 % (w/v) PS20 |
| | 3. Wash 1 | 25 mM Tris/HCl, 25 mM NaCl, pH 7.0 |
| | 4. Wash 2: | 0.04 % (w/v) PS20 (5 CV in total: 4 CV, 30 min hold, 1 CV) |
| | 5. Wash 3 | purified water (type II) |
| | 6. Wash 4 | 25 mM Tris/HCl, 25 mM NaCl, pH 7.0 |
| | 7. Elution | 30 mM Acetic acid (Pool 2.5 AU - 2.5 AU (1 cm UV-cell)) |
| | | |

| Run 5 (conditioned HCCF containing 0.28 % (w/v) PS20): | | |
|---|---|---|
| Load densitiy: | | 19.82 g/Lᵣₑₛᵢₙ |
| Chromatography steps: | | |
| | 1. Equilibration | 25 mM Tris/HCl, 25 mM NaCl, pH 7.0 |
| | 2. Load HCCF | conditioned HCCF containing 0.28 % (w/v) PS20 |
| | 3. Wash 1 | 25 mM Tris/HCl, 25 mM NaCl, pH 7.0 |
| | 4. Wash 2 | purified water (type II) |
| | 5. Wash 3 | 25 mM Tris/HCl, 25 mM NaCl, pH 7.0 |
| | 6. Elution | 30 mM Acetic acid (Pool 2.5 AU - 2.5 AU (1 cm UV-cell)) |
| | | |

| Run 6 (add. wash-strip 0.28 % (w/v) PS20): | | |
|---|---|---|
| Load density: | | 19.81 g/Lᵣₑₛᵢₙ |
| Chromatographic steps: | | |
| | 1. Equilibration | 25 mM Tris/HCl, 25 mM NaCl, pH 7.0 |
| | 2. Load HCCF | |
| | 3. Wash 1 | 25 mM Tris/HCl, 25 mM NaCl, pH 7.0 |
| | 4. Wash 2 | 0.28 % (w/v) PS20 (5 CV in total: 4 CV, 30 min hold, 1 CV) |
| | 5. Wash 3 | purified water (type II) |
| | 6. Wash 4 | 25 mM Tris/HCl, 25 mM NaCl, pH 7.0 |
| | 7. Elution | 30 mM Acetic acid (Pool 2.5 AU - 2.5 AU (1 cm UV-cell)) |
| | | |

| Run 7 (conditioned HCCF containing 0.28 % (w/v) PS20 + add. wash-strip 0.28 % (w/v) PS20) | | |
|---|---|---|
| Load densitiy: | | 19.86 g/Lᵣₑₛᵢₙ |
| Chromatographic steps: | | |
| | 1. Equilibration | 25 mM Tris/HCl, 25 mM NaCl, pH 7.0 |
| | 2. Load HCCF | conditioned HCCF containing 0.28 % (w/v) PS20 |
| | 3. Wash 1 | 25 mM Tris/HCl, 25 mM NaCl, pH 7.0 |
| | 4. Wash 2 | 0.28 % (w/v) PS20 (5 CV in total: 4 CV, 30 min hold, 1 CV) |
| | 5. Wash 3 | purified water (type II) |
| | 6. Wash 4 | 25 mM Tris/HCl, 25 mM NaCl, pH 7.0 |
| | 7. Elution | 30 mM Acetic acid (Pool 2.5 AU - 2.5 AU (1 cm UV-cell)) |

### Anti IgG-Fc (CaptureSelect™ FcXL) run pool summary:

The CaptureSelect™ FcXL eluate (containing obinutuzumab) was adjusted to a pH value of 6,0 ± 0,2 with 1.5 M Tris-amino and consecutively filtered using a 0.45 µm and a 0.22 µm pore size Minisart® filter unit (Sartorius Stedim). Filtered eluate was frozen at -20 °C served as starting material for subsequent analytics.

| **Run** | **PS20 conditioning in HCCF** | **additional PS20 wash-strip** | **Yield Eluate** | **SE-HPLC Main Peak** |
|---|---|---|---|---|
| | **c(PS20) [% (w/v)]** | **c(PS20) [% (w/v)]** | **[%]** | **[%]** |
| 1 | - | - | 82.10 | 98.46 |
| 2 | 0.04 | - | 97.32 | 98.82 |
| 3 | - | 0.04 | 98.72 | 98.79 |
| 4 | 0.04 | 0.04 | 109.05 | 98.78 |
| 5 | 0.28 | - | 102.13 | 98.69 |
| 6 | - | 0.28 | 94.28 | 98.32 |
| 7 | 0.28 | 0.28 | 105.87 | 98.85 |

### Examnle 3

### Purification of an anti-CD20 antibody in a cation exchange chromatography (SP-Sepharose)

### Load preparation:

Following steps were performed sequentially:
1) Two untreated SP loads containing obinutuzumab in different concentrations ("Probe 4.1 Load SPFF"; G002.01E) were thawed and separately filtered using a 0.22 µm pore size filter unit (Merck Millipore).
2) The first SP load fraction was split into three identical portions (used for runs 1-3) and the second SP load was used for run 4. In case of PS20 conditioning, a final concentration of 0.04 % (w/v) PS20 was added to the SP load. The conditioned solutions were homogenized by stirring (300 rpm, 10 min, RT) and incubated for 24 h at 4 °C without stirring. All portions were stored at -20 °C.

### Chromatography:

Chromatographic protein purification was performed using a cation exchange SP Sepharose Fast Flow column (Ø 1 cm, h = 27.3 cm, V = 21.4 ml) (GE Healthcare) on an AEKTA Explorer system (GE Healthcare) and a constant flow rate of 160 cm/h during all steps. Run details are listed as follows:

| Run 1 (Reference): | | |
|---|---|---|
| Load density: | | 51.04 g/Lᵣₑₛᵢₙ |
| Chromatographic steps: | | |
| | 1. Equilibration | 25 mM Tris/Acetate pH 5.0 |
| | 2. Load SPFF | |
| | 3. Wash 1 | 10 mM Tris/Acetate pH 9.0 |
| | 4. Wash 2 | 25 mM Tris/Acetate pH 5.0 |
| | 5. Elution | 25 mM Tris/Acetate, 150 mM NaCl, pH 5.0 (Pool 0.25 AU - 0.88 AU (1 cm UV-cell) |
| | | |

| Run 2 (conditioned SP Load containing 0.04 % (w/v) PS20): | | |
|---|---|---|
| Load Density: | | 49.02 g/Lᵣₑₛᵢₙ |
| Chromatographic steps: | | |
| | 1. Equilibration | 25 mM Tris/Acetate pH 5.0 |
| | 2. Load SPFF | SP Load containing 0.04 % (w/v) PS20 |
| | 3. Wash 1 | 10 mM Tris/Acetate pH 9.0 |
| | 4. Wash 2 | 25 mM Tris/Acetate pH 5.0 |
| | 5. Elution | 25 mM Tris/Acetate, 150 mM NaCl, pH 5.0 (Pool 0.25 AU - 0.88 AU (1 cm UV-cell) |
| | | |

| Run 3 (add. wash-strip 0.04 % (w/v) PS20): | | |
|---|---|---|
| Load density: | | 49.68 g/Lᵣₑₛᵢₙ |
| Chromatographic steps: | | |
| | 1. Equilibration | 25 mM Tris/Acetate pH 5,0 |
| | 2. Load SPFF | |
| | 3. Wash 1 | 10 mM Tris/Acetate pH 9,0 |
| | 4. Wash 2 | 0.04 % (w/v) PS20 (5 CV in total: 4 CV, 30 min hold, 1 CV) |
| | 5. Wash 3 | 25 mM Tris/Acetate pH 5.0 |
| | 6. Elution | 25 mM Tris/Acetate, 150 mM NaCl, pH 5.0 (Pool 0.25 AU - 0.88 AU (1 cm UV-cell) |
| | | |

| Run 4 (conditioned SP Load containing 0.04 % (w/v) PS20 + add. wash-strip 0.04 % (w/v) PS20) | | |
|---|---|---|
| Load density: | | 28.93 g/Lᵣₑₛᵢₙ |
| Chromatography steps: | | |
| | 1. Equilibration | 25 mM Tris/Acetate pH 5.0 |
| | 2. Load SPFF | SP Load containing 0.04 % (w/v) PS20 |
| | 3. Wash 1 | 10 mM Tris/Acetate pH 9.0 |
| | 4. Wash 2 | 0.04 % (w/v) PS20 (5 CV in total: 4 CV, 30 min hold, 1 CV) |
| | 5. Wash 3 | 25 mM Tris/Acetate pH 5.0 |
| | 6. Elution | 25 mM Tris/Acetate, 150 mM NaCl, pH 5.0 (Pool 0.25 AU - 0,88 AU (1 cm UV-cell) |

### SP-Sepharose run pool summary:

SP eluate was frozen at -20 °C and served as starting material for subsequent analytics.

| **Run** | **PS20 conditioning in HCCF** | **additional PS20 wash-strip** | **Yield Eluate** | **SE-HPLC Main Peak** |
|---|---|---|---|---|
| | **c(PS20) [% (w/v)]** | **c(PS20) [% (w/v)]** | **[%]** | **[%]** |
| 1 | - | - | 89.76 | 99.61 |
| 2 | 0.04 | - | 92.62 | 99.13 |
| 3 | - | 0.04 | 90.82 | 99.17 |
| 4 | 0.04 | 0.04 | 83.14 | 98.68 |

### Examnle 4

### Determination of the hydrolytic activity

### Incubation of elution fractions with PS20

To monitor the (remaining) hydrolytic activity in the respective elution fractions from examples 1 to 3, samples were adjusted to the same protein concentration and prepared for PS20 stability studies using a stock solution of PS20 (4.0% (w/v)) and the corresponding elution buffer system as follows. In addition, a stock solution of methionine (100 mM) was added as an efficient antioxidant to control oxidative degradation of PS20 during the time-course of the experiment.

**Protein A (MabSelect SuRe):**

| **Sample ID** | **c (sample) (mL)** | **V (sample) (mL)** | **V (PS20 stock soluti on) (mL)** | **V (methionine stock solution) (mL)** | **V (MSS Elution buffer) (mL)** | **Final protein concentration (mg/mL)** |
|---|---|---|---|---|---|---|
| Reference Elution | 19.89 | 0.603 | 0.050 | 0.500 | 3.847 | 2.40 |
| Elution 0.04% PS20 cond. | 17.04 | 0.704 | 0.050 | 0.500 | 3.746 | 2.40 |
| Elution 0.04% PS20 wash | 18.35 | 0.654 | 0.050 | 0.500 | 3.796 | 2.40 |
| Elution 0.04% PS20 cond./wash | 17.85 | 0.672 | 0.050 | 0.500 | 3.778 | 2.40 |

**anti IgG-Fc (CaptureSelect™ FcXL):**

| **Sample ID** | **c (sample) (mg/mL)** | **V (sample) (mL)** | **V (PS20 stock solution) (mL)** | **V (methionine stock solution) (mL)** | **V (FcXL Elution buffer) (mL)** | **Final protein concentration (mg/mL)** |
|---|---|---|---|---|---|---|
| Reference Elution | 14.550 | 3.093 | 0.050 | 0.500 | 1.357 | 9.00 |
| Elution 0.04% PS20 cond. | 14.067 | 3.199 | 0.050 | 0.500 | 1.251 | 9.00 |
| Elution 0.04% PS20 wash | 16.574 | 2.715 | 0.050 | 0.500 | 1.735 | 9.00 |
| Elution 0.04% PS20 cond./wash | 18.257 | 2.463 | 0.050 | 0.500 | 1.987 | 9.00 |
| Elution 0.28% PS20 cond. | 10.187 | 4.418 | 0.050 | 0.500 | 0.032 | 9.00 |
| Elution 0.28% PS20 wash | 17.186 | 2.618 | 0.050 | 0.500 | 1.832 | 9.00 |
| Elution 0.28% PS20 cond./wash | 15.389 | 2.924 | 0.050 | 0.500 | 1.526 | 9.00 |

**CEX (SP Sepharose):**

| **Sample ID** | **c (sample) (mg/mL)** | **V (sample) (mL)** | **V (PS20 stock solution) (mL)** | **V (methionine stock solution) (mL)** | **V(SP Elution buffer) (mL)** | **Final protein concentration (mg/mL)** |
|---|---|---|---|---|---|---|
| Reference Elution | 9.54 | 2.830 | 0.050 | 0.500 | 1.620 | 5.40 |
| Elution 0.04% PS20 cond. | 9.74 | 2.772 | 0.050 | 0.500 | 1.678 | 5.40 |
| Elution 0.04% PS20 wash | 9.33 | 2.894 | 0.050 | 0.500 | 1.556 | 5.40 |
| Elution 0.04% PS20 cond./wash | 6.07 | 4.448 | 0.050 | 0.500 | 0.002 | 5.40 |

All reaction mixtures were incubated in a Thermomixer at 40°C under shaking at 300 rpm, samples were withdrawn after defined time-points (as indicated in the respective figure descriptions, e.g. at 238 and 317 hours of incubation for Protein A chromatography purification) and stored at -80°C until subsequent analysis

### Quantification of the remaining intact PS 20 content

A method, comparable to those described by Hewitt et al. (Journal of chromatography. A 2011, 1218, 2138-2145) and Lippold et al. (Journal of pharmaceutical and biomedical analysis 2017, 132, 24-34), was used for quantifying the content of PS20. Samples were separated by mixed-mode HPLC using an Oasis MAX Cartridge Column (30 µm, 2.1x20 mm, Waters) and an UltiMate™ 3000 RS (Thermo Fisher) equipped with a LPG-3400XRS Extended Pressure Range Quaternary Pump, an autosampler, a temperature-controlled column compartment and a Corona™ Veo™ RS charged aerosol detector (CAD) (Thermo Fisher). The CAD settings were as follows; internal nitrogen pressure 50 psi provided from an in-house source, power function setting 1.00 and range 200 pA by default. The post column switching valve directed the flow to the CAD between 2.4 and 6.5 min, while diverting the flow to waste at all other times. For analysis, 25 µL of a sample with a nominal PS20 concentration of 0.4 mg/mL was injected and the PS20 content determined by establishing a calibration curve with an injection volume ranging from 5 (2 µg) to 40 µL (16 µg). The column temperature was set to 30°C. Solvent A (2% formic acid in purified water) and solvent B (2% formic acid in methanol) were used as mobile phases for the following gradient at a flow rate of 1.25 mL/min:

| **Time (min)** | **Solvent A** | **Solvent B** | **Switch to** |
|---|---|---|---|
| 0 | 90 | 10 | Waste |
| 1.0 | 60 | 40 | Waste |
| 2.4 | 60 | 40 | Waste |
| 3.4 | 60 | 40 | CAD |
| 3.5 | 0 | 100 | CAD |
| 5.5 | 0 | 100 | CAD |
| 5.6 | 90 | 10 | CAD |
| 6.5 | 90 | 10 | Waste |
| 8-0 | 90 | 10 | Waste |

The obtained PS20 concentration for each sample (in mg/mL) was referred to the corresponding t=0 value (relative PS20 concentration), which represents a defined amount of polysorbate spiked into each sample at the starting point of the experiment. All samples were incubated over a predefined period of time to allow for polysorbate hydrolysis to occur. For graphic representation, the Reference Elution (set to 100%) was put into relation to the relative PS20 concentration in samples treated by the method reported herein (PS20 treatment) (see Figures 2 to 5). The remaining PS 20 content at a given timepoint can thus be correlated with the remaining hydrolytic activity, i.e. the more PS 20 is left within the elution fraction samples (containing the protein of interest along with impurities with hydrolytic activity) after incubation, the less remaining hydrolytic activity is present in the sample, i.e. the reduction of hydrolytically active impurities during purification was more effective. In line with the finding of lower hydrolytic activity in elution fractions after treatment with polysorbate, also higher hydrolytic activity could be detected in the respective wash fractions.

Supplementary analyses have been made to detect free fatty acids resulting from the hydrolytic degradation of PS. As for the results shown for remaining PS content, the results showed that a lower amount of free fatty acids (degradation product) could be detected in samples where a treatment with polysorbate in the purification was performed.

It can be observed that the degree of purification is most distinct if the solution comprising the protein is incubated/conditioned with a polysorbate before the chromatographic step and in addition the solution comprising the protein is also washed with a solution comprising a polysorbate during the chromatography. The combination of those treatment leads to even better results than the single treatments alone.

## Claims

1. A method for purifying a protein from a sample comprising the protein and at least one impurity with hydrolytic activity, comprising the following steps:
i)
a) applying the sample comprising the protein and at least one impurity with hydrolytic activity to a chromatography material,
b) applying a solution which comprises a polysorbate to the chromatography material, and
c) recovering the protein from the chromatography material, or
ii)
a) adding a polysorbate to the sample comprising the protein and at least one impurity with hydrolytic activity,
b) applying the mixture of step a) to a chromatography material, and
c) recovering the protein from the chromatography material, or
iii)
a) adding a polysorbate to the sample comprising the protein and at least one impurity with hydrolytic activity,
b) applying the mixture of step a) to a chromatography material,
c) applying a solution which comprises a polysorbate to the chromatography material, and
d) recovering the protein from the chromatography material.

2. A method for reducing the hydrolytic activity in a sample comprising a protein and at least one impurity with hydrolytic activity, comprising the following steps:
i)
a) applying the sample comprising the protein and at least one impurity with hydrolytic activity to a chromatography material,
b) applying a solution which comprises a polysorbate to the chromatography material, and
c) recovering the protein from the chromatography material, or
ii)
a) adding a polysorbate to the sample comprising the protein and at least one impurity with hydrolytic activity,
b) applying the mixture of step a) to a chromatography material, and
c) recovering the protein from the chromatography material, or
iii)
a) adding a polysorbate to the sample comprising the protein and at least one impurity with hydrolytic activity,
b) applying the mixture of step a) to a chromatography material,
c) applying a solution which comprises a polysorbate to the chromatography material, and
d) recovering the protein from the chromatography material.

3. The method according to any one of claims 1 to 2, wherein the impurity is a hydrolase.

4. The method according to any one of claims 1 to 3, wherein the impurity is an esterase.

5. The method according to any one of claims 1 to 4, wherein the polysorbate is selected from the group comprising polysorbate 20, polysorbate 40, polysorbate 60, polysorbate 65 or polysorbate 80.

6. The method according to any one of claims 1 to 5, wherein the polysorbate is polysorbate 20.

7. The method according to any one of claims 1 to 6, wherein the polysorbate is added to a final concentration of at least about 0.001% (w/v).

8. The method according to any one of claims 1 to 7, wherein the polysorbate is added to a final concentration of from about 0.001% (w/v) to about 10% (w/v).

9. The method according to any one of claims 1 to 8, wherein the chromatography material is an affinity chromatography material, a cation exchange chromatography material, an anion exchange chromatography material, a hydrophobic interaction chromatography material or a mixed mode chromatography material.

10. The method according to any one of claims 1 to 9, wherein the chromatography material is an affinity chromatography material or a cation exchange chromatography material.

11. The method according to any one of claims 1 to 10, wherein the chromatography material is an affinity chromatography material selected from Protein A chromatography or a chromatography material with a camelid-derived single domain antibody fragment as ligand.

12. The method according to any one of claims 1 to 11, wherein the affinity chromatography material is CaptureSelect FcXL or MabSelect SuRe.

13. The method according to any one of claims 1 to 10, wherein the cation exchange chromatography material is SP Sepharose Fast Flow.

14. The method according to any one of claims 1 to 13, wherein the protein is a monoclonal antibody.

15. The method according to any one of claims 1 to 14, wherein the protein is a humanized Type II anti-CD20 antibody.

16. A liquid antibody preparation with reduced hydrolytic activity obtained by a method according to any one of the preceding claims.

17. A method for producing a protein comprising the following steps:
a) cultivating a mammalian cell comprising a nucleic acid encoding the protein,
b) recovering the protein from the cell or the cultivation medium, and
c) purifying the protein with a method according to any one of claims 1 to 15,
and thereby producing the protein.


SEQUENCE LISTING

<110>  F. Hoffmann-La Roche AG

<120>  Methods for purification of polypeptides using polysorbates

<130>  P34772-EP

<160>  2

<170>  PatentIn version 3.5

<210>  1
<211>  119
<212>  PRT
<213>  Artificial

<220>
<223>  amino acid sequences of variable region of the heavy chain (VH)
       of humanized B-Ly1 antibody (B-HH6)

<400>  1

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ala Phe Ser Tyr Ser
            20                  25                  30

Trp Ile Asn Trp Val Arg Gln Ala Pro Gly Gln Gly Leu Glu Trp Met
        35                  40                  45

Gly Arg Ile Phe Pro Gly Asp Gly Asp Thr Asp Tyr Asn Gly Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
            100                 105                 110

Thr Leu Val Thr Val Ser Ser
        115

<210>  2
<211>  115
<212>  PRT
<213>  Artificial

<220>
<223>  amino acid sequences of variable region of the light chain (VL)
       of humanized B-Ly1 antibody B-KV1

<400>  2

Asp Ile Val Met Thr Gln Thr Pro Leu Ser Leu Pro Val Thr Pro Gly
1               5                   10                  15

Glu Pro Ala Ser Ile Ser Cys Arg Ser Ser Lys Ser Leu Leu His Ser
            20                  25                  30

Asn Gly Ile Thr Tyr Leu Tyr Trp Tyr Leu Gln Lys Pro Gly Gln Ser
        35                  40                  45

Pro Gln Leu Leu Ile Tyr Gln Met Ser Asn Leu Val Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala Gln Asn
                85                  90                  95

Leu Glu Leu Pro Tyr Thr Phe Gly Gly Gly Thr Lys Val Glu Ile Lys
            100                 105                 110

Arg Thr Val
        115